# EUROPEAN PATENT APPLICATION

(11) **EP 2 405 268 A1**
(43) Date of publication of application: **11.01.2012**
(21) Application number: 10748752.2
(22) Date of filing: 02.03.2010
(51) Int. Cl.: G01N 33/543, G01N 33/53, C12N 15/09

(54) **METHOD FOR DETECTING SUBSTANCE IN BIOLOGICAL SAMPLE**

(30) Priority: 02.03.2009 JP 2009048661
(71) Applicant: Japan Tobacco, Inc., Tokyo 105-8422 (JP); Virus Ikagaku Kenkyusho Inc., Osaka 560-0082 (JP)
(72) Inventor: TAKAKURA, Yoshimitsu, Iwata-shi Shizuoka 438-0802 (JP); OKA, Naomi, Iwata-shi Shizuoka 438-0802 (JP); KONDO, Kazuhiro, Tokyo 105-8461 (JP)
(74) Representative: Reinhard - Skuhra - Weise & Partner GbR
(86) International application number: PCT/JP2010/053359
(87) International publication number: WO 2010/101157

(57) **Abstract**

The present invention provides a method for detecting a substance in a biological sample, a carrier for using in the method, and a kit. The method of the present invention according to an embodiment includes:
1) providing a carrier on which biotin is bound and providing a fusion protein between a protein that specifically binds to a substance to be detected and a biotin-binding protein;
2) binding the fusion protein to the carrier provided in step 1) through binding between biotin and the biotin-binding protein to produce a fusion protein-bound carrier;
3) mixing
(a) a biological sample, and
(b-i) a cell homogenate extract prepared from cells of the same species as the host cells used for expressing the fusion protein in step 1), and a biotin-binding protein, or
(b-ii) a cell homogenate extract prepared from cells of the same species as the host cells used for expressing the fusion protein in step 1), and genetically engineered to express a biotin-binding protein,

and adding the mixture to the fusion protein-bound carrier produced in step 2); and
4) detecting a substance that has bound to the protein that specifically binds to a substance to be detected in the fusion protein.

SEQUENCE LISTING

<110>  Virus Ikagaku kenkyusho Inc.
       Japan Tobacco Inc.

<120>  Method for detecting substance in biological sample

<130>  P31980-WOEP

<140>  EP 10 748 752.2
<141>  2010-03-02

<150>  PCT/JP2010/053359
<151>  2010-03-02

<160>  31

<170>  PatentIn version 3.1

<210>  1
<211>  159
<212>  PRT
<213>  Human herpesvirus 6

<400>  1

Met Gly Tyr Glu Glu Lys Val Ser Ala Thr Gly Lys Thr Arg Leu Lys
1               5                   10                  15

Ile Leu Ala Cys Leu Ile Val Leu Ile Leu Ala Ala Ala Ile Thr Met
            20                  25                  30

Leu Thr Leu Glu Ile Ile Ser Asn Gln Lys Arg Thr Thr Thr Asp Leu
        35                  40                  45

Glu Ala Val Thr Val Ala Leu Lys His Val Ser Thr Ser Leu Ala Ser
    50                  55                  60

Cys Thr Glu Ser Thr Thr Ser Val His Thr Asp Ser Val Thr Ser Gln
65                  70                  75                  80

Pro Thr Lys Asn Lys Glu Ser Arg Lys Lys Ile Glu Gly Lys Ser Pro
                85                  90                  95

Ser Trp Val Gln Ala Leu Thr Thr Ala Ser Gly Ile Ile Leu Leu Phe
            100                 105                 110

Cys Ile Met Met Ile Phe Ile Thr Cys Ser Trp Thr Thr Glu Lys Asp
        115                 120                 125

Thr Glu Lys Ser Glu Val Gln Ser Tyr Ala Ser Ser Val Glu Thr Leu
    130                 135                 140

Asp Ser Leu Asn Glu Ala Ile Ile Pro Lys Thr Glu Met Asn Val
145                 150                 155


<210>  2
<211>  480
<212>  DNA

<213>  Human herpesvirus 6

<400>  2
atgggatatg aagaaaaagt gtcagctact ggaaagactc gtttaaagat actggcatgt      60
ctgatcgttt taatactagc tgcggcaata actatgttaa cgctggaaat tatatcgaac     120
caaaaacgta ccactactga tctcgaagct gtgactgtgg cgctgaagca tgtaagcaca     180
tctcttgcca gctgcactga atccactact tctgtacata ccgattctgt gacgagccaa     240
cccacgaaaa acaaagaatc gaggaaaaaa attgaaggga aatctccaag ttgggttcag     300
gctttaacta cagcatctgg aattatccta ctgttttgta taatgatgat attcattaca     360
tgttcctgga ccacagaaaa agatacagag aagagtgaag tgcaatctta tgcttcttca     420
gtagagactt tagactcttt aaatgaggct attataccga aaactgaaat gaatgtgtaa     480


<210>  3
<211>  1795
<212>  DNA
<213>  Human herpesvirus 6

<400>  3
aggctctgct ggaggctctg ctggaggcct tgctgaaggc tctgctggag gccctgctgg      60
aggtcttgct ggaggctctg ctggaggctc tgctggaggc tctgctggag gctctgctgg     120
aggctctgct ggaggctctg ctggaggctc tgtcagagac ctcggtgaaa gttttactca     180
gaggtttatc agagttttcg ccattagttt ggttagaagt ttcagattta ttttcggtgg     240
aactgcagtt aggtttcatg tcagtacatt catcaccgtt agaagtgcta ttcatggtgc     300
tgttgccact gttggatttg ttaaaagcag taaatgagct aggattggaa tgactccgaa     360
tagctaataa atttgagcat tttcttcgaa tggatcataa tcagagggat agccatctaa     420
tttaaagact tccattttat cactgttgca atcacttcta atggagtatc tggatacatt     480
ttttctacat ctttttcatc ccctccaaca tggatctgtg cagcgttaat aagccagcgg     540
agttaattaa atcgtcttcc atgttagaca gttcctgttt catggcagcc ttcactgatg     600
caccaatact ttggatgcaa gtgccaacgg actgagctag gatgtaaaag aagatattct     660
aattttgaat tcttcagatg ctccttcttc cacattactg gaataggaca cattcttgga     720
agcgatgtcg ttggaagact ctgggatgaa aagatcacag gcttccagtt ctggaaaaag     780
caggctttca aaggacacat cacacttgag actctcttcc aatatttctt tgatggattc     840
ttccaccact ggatcgggat ggtagctata tatactatat aaggagatta ccaccaccac     900
ctctttcttt gcagagatta ttctctgctt gaaaatctgt aacactgatc atgatgggat     960
atgaagaaaa agtgtcagct actggaaaga ctcgtttaaa gatactggca tgtctgatcg    1020
ttttaatact agctgcggca ataactatgt taacgctgga aattatatcg aaccaaaaac    1080
gtaccactac tgatctcgaa gctgtgactg tggcgctgaa gcatgtaagc acatctcttg    1140
ccagctgcac tgaatccact acttctgtac ataccgattc tgtgacgagc caacccacga    1200
aaaacaaaga atcgaggaaa aaaattgaag ggaaatctcc aagttgggtt caggctttaa    1260
ctacagcatc tggaattatc ctactgtttt gtataatgat gatattcatt acatgtccct    1320
ggaccacaga aaaagataca gagaagagtg aagtgcaatc ttatgctcct tcagtagaga    1380
ctttagaccc tttaaatgag gctattatac cgaaaactga aatgaatgtg taatgtctgt    1440
atttttcttt acagagatgt acggagagtt tatatttggg gaaaatacct gactgttctg    1500
cctatatgcg aatgttaaag tatgtataat ataaattctt accttttaag agtgattcaa    1560
ggtggaggtt tctttggaga ttgattccag gtggtggttt cgggtgcaat caatctttct    1620
tctgggcggg aagaaaatcc agcaatccaa taattgatgg gatgtaatca atgtcacaaa    1680
tctgtaagat taaatgtgaa cagtataaat tctttcgtgc ttatcaaatt acaattatgc    1740
gcatgaaaat atcattaaat tgttttaaac attcttaaaa aaaaaaaaaa aaaaa         1795

<210>  4
<211>  432
<212>  DNA
<213>  Pleurotus cornucopiae

<220>
<221>  CDS
<222>  (1)..(432)

<400>  4

atg aaa gac gtc caa tct ctc ctc acc gga acc tgg tac aat gaa ctc 48
Met Lys Asp Val Gln Ser Leu Leu Thr Gly Thr Trp Tyr Asn Glu Leu
1               5                   10                  15

ggc tca aca atg aat ttg act gca aat aaa gac ggt tcg ctc acc gga 96
Gly Ser Thr Met Asn Leu Thr Ala Asn Lys Asp Gly Ser Leu Thr Gly
            20                  25                  30

acg tac cac tcc aac gtc ggc gag gtt ccc cca act tat cac ctt tct 144
Thr Tyr His Ser Asn Val Gly Glu Val Pro Pro Thr Tyr His Leu Ser
        35                  40                  45

ggc cgg tac aac ctc cag ccc ccc tcg ggt caa ggc gtt act ctg gga 192
Gly Arg Tyr Asn Leu Gln Pro Pro Ser Gly Gln Gly Val Thr Leu Gly
    50                  55                  60

tgg gcg gtg tct ttc gaa aac act agt gcg aat gtt cat tct gtc tca 240
Trp Ala Val Ser Phe Glu Asn Thr Ser Ala Asn Val His Ser Val Ser
65                  70                  75                  80

aca tgg agc ggg cag tac ttc tct gaa ccc gcc gag gtg atc ctc acc 288
Thr Trp Ser Gly Gln Tyr Phe Ser Glu Pro Ala Glu Val Ile Leu Thr
                85                  90                  95

cag tgg ctg ttg tca agg agc tct gag cgc gaa gat ttg tgg cag tcc 336
Gln Trp Leu Leu Ser Arg Ser Ser Glu Arg Glu Asp Leu Trp Gln Ser
            100                 105                 110

acc cat gtg ggg cat gat gag ttc agc aag aca aag cca acc aag gag 384
Thr His Val Gly His Asp Glu Phe Ser Lys Thr Lys Pro Thr Lys Glu
        115                 120                 125

aag att gcc cag gct caa ctc ctt cgt cgc ggg ttg aag ttc gag tga 432
Lys Ile Ala Gln Ala Gln Leu Leu Arg Arg Gly Leu Lys Phe Glu
    130                 135                 140

<210> 5
<211> 143
<212> PRT
<213> Pleurotus cornucopiae

<400> 5
Met Lys Asp Val Gln Ser Leu Leu Thr Gly Thr Trp Tyr Asn Glu Leu
1               5                   10                  15

Gly Ser Thr Met Asn Leu Thr Ala Asn Lys Asp Gly Ser Leu Thr Gly
            20                  25                  30

Thr Tyr His Ser Asn Val Gly Glu Val Pro Pro Thr Tyr His Leu Ser
        35                  40                  45

Gly Arg Tyr Asn Leu Gln Pro Pro Ser Gly Gln Gly Val Thr Leu Gly
    50                  55                  60

Trp Ala Val Ser Phe Glu Asn Thr Ser Ala Asn Val His Ser Val Ser
65                  70                  75                  80

Thr Trp Ser Gly Gln Tyr Phe Ser Glu Pro Ala Glu Val Ile Leu Thr
                85                  90                  95

Gln Trp Leu Leu Ser Arg Ser Ser Glu Arg Glu Asp Leu Trp Gln Ser
            100                 105                 110

Thr His Val Gly His Asp Glu Phe Ser Lys Thr Lys Pro Thr Lys Glu
        115                 120                 125

Lys Ile Ala Gln Ala Gln Leu Leu Arg Arg Gly Leu Lys Phe Glu
    130                 135                 140

<210>  6
<211>  426
<212>  DNA
<213>  Pleurotus cornucopiae

<220>
<221>  CDS
<222>  (1)..(426)

<400>  6
atg tca gac gtt caa tct tca ctc acc gga acc tgg tac aat gaa ctc       48
Met Ser Asp Val Gln Ser Ser Leu Thr Gly Thr Trp Tyr Asn Glu Leu
1               5                   10                  15

aac tcc aag atg gaa ttg act gca aac aaa gac ggt act ctc act gga       96
Asn Ser Lys Met Glu Leu Thr Ala Asn Lys Asp Gly Thr Leu Thr Gly
            20                  25                  30

aag tac ctc tcc aaa gtt ggg gat gtc tac gtg ccc tac cca ctc tct      144
Lys Tyr Leu Ser Lys Val Gly Asp Val Tyr Val Pro Tyr Pro Leu Ser
        35                  40                  45

ggt cgc tat aac ctc caa ccc ccc gcg gga caa ggc gtc gct ctt ggg      192
Gly Arg Tyr Asn Leu Gln Pro Pro Ala Gly Gln Gly Val Ala Leu Gly
    50                  55                  60

tgg gcg gta tcc tgg gag aac agt aaa att cat tcc gct acg aca tgg      240
Trp Ala Val Ser Trp Glu Asn Ser Lys Ile His Ser Ala Thr Thr Trp
65                  70                  75                  80

agc gga cag ttc ttc tct gag tcg tct cca gtg att ctt act cag tgg      288
Ser Gly Gln Phe Phe Ser Glu Ser Ser Pro Val Ile Leu Thr Gln Trp
                85                  90                  95

ttg ttg tca tcg agc act gcg cgt ggg gac gta tgg gaa tcc aca ctt      336
Leu Leu Ser Ser Ser Thr Ala Arg Gly Asp Val Trp Glu Ser Thr Leu
            100                 105                 110

gtg ggg aat gat tcg ttt aca aag acg gcg ccg act gag cag cag atc      384
Val Gly Asn Asp Ser Phe Thr Lys Thr Ala Pro Thr Glu Gln Gln Ile
        115                 120                 125

gct cat gct caa ctc cat tgt cgc gca ccg agg ttg aag taa              426
Ala His Ala Gln Leu His Cys Arg Ala Pro Arg Leu Lys
    130                 135                 140


<210>  7
<211>  141
<212>  PRT

<213>  Pleurotus cornucopiae

<400>  7
Met Ser Asp Val Gln Ser Ser Leu Thr Gly Thr Trp Tyr Asn Glu Leu
1               5                   10                  15

Asn Ser Lys Met Glu Leu Thr Ala Asn Lys Asp Gly Thr Leu Thr Gly
            20                  25                  30

Lys Tyr Leu Ser Lys Val Gly Asp Val Tyr Val Pro Tyr Pro Leu Ser
        35                  40                  45

Gly Arg Tyr Asn Leu Gln Pro Pro Ala Gly Gln Gly Val Ala Leu Gly
    50                  55                  60

Trp Ala Val Ser Trp Glu Asn Ser Lys Ile His Ser Ala Thr Thr Trp
65                  70                  75                  80

Ser Gly Gln Phe Phe Ser Glu Ser Ser Pro Val Ile Leu Thr Gln Trp
                85                  90                  95

Leu Leu Ser Ser Ser Thr Ala Arg Gly Asp Val Trp Glu Ser Thr Leu
            100                 105                 110

Val Gly Asn Asp Ser Phe Thr Lys Thr Ala Pro Thr Glu Gln Gln Ile
        115                 120                 125

Ala His Ala Gln Leu His Cys Arg Ala Pro Arg Leu Lys
    130                 135                 140

<210>  8
<211>  5
<212>  PRT
<213>  Artificial Sequence

<220>
<223>  Peptide linker

<400>  8
Gly Gly Gly Gly Ser
1               5

<210>  9
<211>  5
<212>  PRT
<213>  Artificial Sequence

<220>
<223>  Peptide linker

<400>  9
Gly Gly Ser Gly Gly
1               5

<210>  10
<211>  5
<212>  PRT
<213>  Artificial Sequence

<220>

<223>  Peptide linker

<400>  10
Gly Ala Ser Ala Gly
1               5

<210>  11
<211>  5
<212>  PRT
<213>  Artificial Sequence

<220>
<223>  Peptide linker

<400>  11
Gly Ser Gly Ala Ala
1               5

<210>  12
<211>  5
<212>  PRT
<213>  Artificial Sequence

<220>
<223>  Peptide linker

<400>  12
Gly Ser Gly Ser Ala
1               5

<210>  13
<211>  6
<212>  PRT
<213>  Artificial Sequence

<220>
<223>  Peptide linker

<400>  13
Gly Gly Gly Gly Ser Gly
1               5   6

<210>  14
<211>  7
<212>  PRT
<213>  Artificial Sequence

<220>
<223>  Peptide linker

<400>  14
Gly Gly Gly Ser Gly Gly Ser
1               5       7

<210>  15
<211>  8
<212>  PRT
<213>  Artificial Sequence

<220>

<223>  Peptide linker

<400>  15
Gly Gly Ser Gly Gly Gly Gly Ser
1               5           8

<210>  16
<211>  9
<212>  PRT
<213>  Artificial Sequence

<220>
<223>  Peptide linker

<400>  16
Ala Ala Ala Ala Gly Ser Gly Ala Ala
1               5               9

<210>  17
<211>  15
<212>  PRT
<213>  Artificial Sequence

<220>
<223>  Peptide linker

<400>  17
Gly Gly Gly Gly Ser Gly Gly Gly Gly Ser Gly Gly Gly Gly Ser
1               5                   10                  15

<210>  18
<211>  25
<212>  PRT
<213>  Artificial Sequence

<220>
<223>  Peptide linker

<400>  18
Gly Gly Gly Gly Ser Gly Gly Gly Gly Ser Gly Gly Gly Gly Ser Gly
1               5                   10                  15
Gly Gly Gly Ser Gly Gly Gly Gly Ser
        20                  25

<210>  19
<211>  117
<212>  DNA
<213>  Artificial Sequence

<220>
<223>  primer SITH1C-5xlink-TM2N-F

<400>  19
ccgaaaactg aaatgaatgt gggtggcggt ggcagcggtg gcggtggcag cggtggcggt    60
ggcagcggtg gcggtggcag cggtggcggt ggcagctcag acgttcaatc ttcactc      117

<210>  20
<211>  117
<212>  DNA
<213>  Artificial Sequence

<220>
<223>  primer SITH1C-5xlink-TM2N-R

<400>  20
gagtgaagat tgaacgtctg agctgccacc gccaccgctg ccaccgccac cgctgccacc    60
gccaccgctg ccaccgccac cgctgccacc gccacccaca ttcatttcag ttttcgg      117

<210>  21
<211>  29
<212>  DNA
<213>  Artificial Sequence

<220>
<223>  primer SITH1C-5′ EcoRI-F

<400>  21
aaagaattcg gatatgaaga aaaagtgtc     29

<210>  22
<211>  28
<212>  DNA
<213>  Artificial Sequence

<220>
<223>  primer TM2CtermBam

<400>  22
tttggatcct tacttcaacc tcggtgcg      28

<210>  23
<211>  981
<212>  DNA
<213>  Artificial Sequence

<220>
<223>  DNA encoding fusion protein SITH-1-TM2

<400>  23
atg gaa ttc gga tat gaa gaa aaa gtg tca gct act gga aag act cgt
48
Met Glu Phe Gly Tyr Glu Glu Lys Val Ser Ala Thr Gly Lys Thr Arg
  1               5                  10                  15

tta aag ata ctg gca tgt ctg atc gtt tta ata cta gct gcg gca ata
96
Leu Lys Ile Leu Ala Cys Leu Ile Val Leu Ile Leu Ala Ala Ala Ile
             20                  25                  30

act atg tta acg ctg gaa att ata tcg aac caa aaa cgt acc act act
144
Thr Met Leu Thr Leu Glu Ile Ile Ser Asn Gln Lys Arg Thr Thr Thr
         35                  40                  45

gat ctc gaa gct gtg act gtg gcg ctg aag cat gta agc aca tct ctt
192
Asp Leu Glu Ala Val Thr Val Ala Leu Lys His Val Ser Thr Ser Leu
     50                  55                  60

gcc agc tgc act gaa tcc act act tct gta cat acc gat tct gtg acg

240
Ala Ser Cys Thr Glu Ser Thr Thr Ser Val His Thr Asp Ser Val Thr
65                  70                  75                  80

agc caa ccc acg aaa aac aaa gaa tcg agg aaa aaa att gaa ggg aaa
288
Ser Gln Pro Thr Lys Asn Lys Glu Ser Arg Lys Lys Ile Glu Gly Lys
                85                  90                  95

tct cca agt tgg gtt cag gct tta act aca gca tct gga att atc cta
336
Ser Pro Ser Trp Val Gln Ala Leu Thr Thr Ala Ser Gly Ile Ile Leu
            100                 105                 110

ctg ttt tgt ata atg atg ata ttc att aca tgt tcc tgg acc aca gaa
384
Leu Phe Cys Ile Met Met Ile Phe Ile Thr Cys Ser Trp Thr Thr Glu
        115                 120                 125

aaa gat aca gag aag agt gaa gtg caa tct tat gct tct tca gta gag
432
Lys Asp Thr Glu Lys Ser Glu Val Gln Ser Tyr Ala Ser Ser Val Glu
    130                 135                 140

act tta gac tct tta aat gag gct att ata ccg aaa act gaa atg aat
480
Thr Leu Asp Ser Leu Asn Glu Ala Ile Ile Pro Lys Thr Glu Met Asn
145                 150                 155                 160

gtg ggt ggc ggt ggc agc ggt ggc ggt ggc agc ggt ggc ggt ggc agc
528
Val Gly Gly Gly Gly Ser Gly Gly Gly Gly Ser Gly Gly Gly Gly Ser
                165                 170                 175

ggt ggc ggt ggc agc ggt ggc ggt ggc agc tca gac gtt caa tct tca
576
Gly Gly Gly Gly Ser Gly Gly Gly Gly Ser Ser Asp Val Gln Ser Ser
            180                 185                 190

ctc acc gga acc tgg tac aat gaa ctc aac tcc aag atg gaa ttg act
624
Leu Thr Gly Thr Trp Tyr Asn Glu Leu Asn Ser Lys Met Glu Leu Thr
        195                 200                 205

gca aac aaa gac ggt act ctc act gga aag tac ctc tcc aaa gtt ggg
672
Ala Asn Lys Asp Gly Thr Leu Thr Gly Lys Tyr Leu Ser Lys Val Gly
    210                 215                 220

gat gtc tac gtg ccc tac cca ctc tct ggt cgc tat aac ctc caa ccc
720
Asp Val Tyr Val Pro Tyr Pro Leu Ser Gly Arg Tyr Asn Leu Gln Pro
225                 230                 235                 240

ccc gcg gga caa ggc gtc gct ctt ggg tgg gcg gta tcc tgg gag aac
768
Pro Ala Gly Gln Gly Val Ala Leu Gly Trp Ala Val Ser Trp Glu Asn
                245                 250                 255

agt aaa att cat tcc gct acg aca tgg agc gga cag ttc ttc tct gag

816
Ser Lys Ile His Ser Ala Thr Thr Trp Ser Gly Gln Phe Phe Ser Glu
            260                 265                 270

tcg tct cca gtg att ctt act cag tgg ttg ttg tca tcg agc act gcg
864
Ser Ser Pro Val Ile Leu Thr Gln Trp Leu Leu Ser Ser Ser Thr Ala
        275                 280                 285

cgt ggg gac gta tgg gaa tcc aca ctt gtg ggg aat gat tcg ttt aca
912
Arg Gly Asp Val Trp Glu Ser Thr Leu Val Gly Asn Asp Ser Phe Thr
    290                 295                 300

aag acg gcg ccg act gag cag cag atc gct cat gct caa ctc cat tgt
960
Lys Thr Ala Pro Thr Glu Gln Gln Ile Ala His Ala Gln Leu His Cys
305                 310                 315                 320

cgc gca ccg agg ttg aag taa
981
Arg Ala Pro Arg Leu Lys
                325

<210>  24
<211>  326
<212>  PRT
<213>  Artificial Sequence

<220>
<223>  Fusion protein SITH-1-TM2

<400>  24

Met Glu Phe Gly Tyr Glu Glu Lys Val Ser Ala Thr Gly Lys Thr Arg
  1               5                  10                  15
Leu Lys Ile Leu Ala Cys Leu Ile Val Leu Ile Leu Ala Ala Ala Ile
            20                  25                  30
Thr Met Leu Thr Leu Glu Ile Ile Ser Asn Gln Lys Arg Thr Thr Thr
        35                  40                  45
Asp Leu Glu Ala Val Thr Val Ala Leu Lys His Val Ser Thr Ser Leu
    50                  55                  60
Ala Ser Cys Thr Glu Ser Thr Thr Ser Val His Thr Asp Ser Val Thr
 65                  70                  75                  80
Ser Gln Pro Thr Lys Asn Lys Glu Ser Arg Lys Lys Ile Glu Gly Lys
                85                  90                  95
Ser Pro Ser Trp Val Gln Ala Leu Thr Thr Ala Ser Gly Ile Ile Leu
            100                 105                 110
Leu Phe Cys Ile Met Met Ile Phe Ile Thr Cys Ser Trp Thr Thr Glu
        115                 120                 125
Lys Asp Thr Glu Lys Ser Glu Val Gln Ser Tyr Ala Ser Ser Val Glu
    130                 135                 140
Thr Leu Asp Ser Leu Asn Glu Ala Ile Ile Pro Lys Thr Glu Met Asn
145                 150                 155                 160
Val Gly Gly Gly Gly Ser Gly Gly Gly Gly Ser Gly Gly Gly Gly Ser
                165                 170                 175
Gly Gly Gly Gly Ser Gly Gly Gly Gly Ser Ser Asp Val Gln Ser Ser
            180                 185                 190
Leu Thr Gly Thr Trp Tyr Asn Glu Leu Asn Ser Lys Met Glu Leu Thr
        195                 200                 205

Ala Asn Lys Asp Gly Thr Leu Thr Gly Lys Tyr Leu Ser Lys Val Gly
    210                 215                 220
Asp Val Tyr Val Pro Tyr Pro Leu Ser Gly Arg Tyr Asn Leu Gln Pro
225                 230                 235                 240
Pro Ala Gly Gln Gly Val Ala Leu Gly Trp Ala Val Ser Trp Glu Asn
                245                 250                 255
Ser Lys Ile His Ser Ala Thr Thr Trp Ser Gly Gln Phe Phe Ser Glu
            260                 265                 270
Ser Ser Pro Val Ile Leu Thr Gln Trp Leu Leu Ser Ser Ser Thr Ala
        275                 280                 285
Arg Gly Asp Val Trp Glu Ser Thr Leu Val Gly Asn Asp Ser Phe Thr
    290                 295                 300
Lys Thr Ala Pro Thr Glu Gln Gln Ile Ala His Ala Gln Leu His Cys
305                 310                 315                 320
Arg Ala Pro Arg Leu Lys
                325

<210>  25
<211>  29
<212>  DNA
<213>  Artificial Sequence

<220>
<223>  primer HarpinNtermEcoRI-F

<400>  25
aaagaattca tgcagagtct cagtcttaa      29

<210>  26
<211>  114
<212>  DNA
<213>  Artificial Sequence

<220>
<223>  primer HarpinC-5xlink-TM2N-R

<400>  26
gtgaagattg aacgtctgag ctgccaccgc caccgctgcc accgccaccg ctgccaccgc    60
caccgctgcc accgccaccg ctgccaccgc caccggctgc agcctgattg cggg          114

<210>  27
<211>  114
<212>  DNA
<213>  Artificial Sequence

<220>
<223>  primer HarpinC-5xlink-TM2N-F

<400>  27
cccgcaatca ggctgcagcc ggtggcggtg gcagcggtgg cggtggcagc ggtggcggtg    60
gcagcggtgg cggtggcagc ggtggcggtg gcagctcaga cgttcaatct tcac          114

<210>  28
<211>  1029
<212>  DNA
<213>  Pseudomonas syringae

<220>
<221>  CDS
<222>  (1)..(1029)

<400>  28
atg cag agt ctc agt ctt aac agc agc tcg ctg caa acc ccg gca atg
48
Met Gln Ser Leu Ser Leu Asn Ser Ser Ser Leu Gln Thr Pro Ala Met
1               5                   10                  15

gcc ctt gtc ctg gta cgt cct gaa acc gag acg act ggc gcc agt acg
96
Ala Leu Val Leu Val Arg Pro Glu Thr Glu Thr Thr Gly Ala Ser Thr
            20                  25                  30

tcg agc aag gcg ctt cag gaa gtt gtc gtg aag ctg gcc gag gaa ctg
144
Ser Ser Lys Ala Leu Gln Glu Val Val Val Lys Leu Ala Glu Glu Leu
        35                  40                  45

atg cgc aat ggt caa ctc gac gac agc tcg cca ttg ggc aaa ctg ctg
192
Met Arg Asn Gly Gln Leu Asp Asp Ser Ser Pro Leu Gly Lys Leu Leu
    50                  55                  60

gcc aag tcg atg gcc gcg gat ggc aag gca ggc ggc ggt atc gag gat
240
Ala Lys Ser Met Ala Ala Asp Gly Lys Ala Gly Gly Gly Ile Glu Asp
65                  70                  75                  80

gtc atc gct gcg ctg gac aag ctg att cat gaa aag ctg ggt gac aac
288
Val Ile Ala Ala Leu Asp Lys Leu Ile His Glu Lys Leu Gly Asp Asn
                85                  90                  95

ttc ggc gcg tct gcg gac aac gcc tcg ggt acc gga cag cag gac ctg
336
Phe Gly Ala Ser Ala Asp Asn Ala Ser Gly Thr Gly Gln Gln Asp Leu
            100                 105                 110

atg act cag gtg ctc agt ggc ctg gcc aag tct atg ctc gat gat ctt
384
Met Thr Gln Val Leu Ser Gly Leu Ala Lys Ser Met Leu Asp Asp Leu
        115                 120                 125

ctg acc aag cag gat ggc ggg gca agc ttc tcc gaa gac gat atg ccg
432
Leu Thr Lys Gln Asp Gly Gly Ala Ser Phe Ser Glu Asp Asp Met Pro
    130                 135                 140

atg ctg aac aag atc gcg cag ttc atg gat gac aat ccc gca cag ttt
480
Met Leu Asn Lys Ile Ala Gln Phe Met Asp Asp Asn Pro Ala Gln Phe
145                 150                 155                 160

ccc aag ccg gac tcg ggt tcc tgg gtg aac gaa ctc aag gaa gac aac
528
Pro Lys Pro Asp Ser Gly Ser Trp Val Asn Glu Leu Lys Glu Asp Asn
                165                 170                 175

ttc ctt gat ggc gac gaa acg gct gcg ttc cgc tcg gca ctc gac atc
576
Phe Leu Asp Gly Asp Glu Thr Ala Ala Phe Arg Ser Ala Leu Asp Ile

180                 185                 190

att ggc cag caa ctg ggt aat cag cag agt ggc gct ggc ggt ctg gcg
624
Ile Gly Gln Gln Leu Gly Asn Gln Gln Ser Gly Ala Gly Gly Leu Ala
        195                 200                 205

ggg acg ggt gga ggt ctg ggc act ccg agc agt ttt tct aac aac tcg
672
Gly Thr Gly Gly Gly Leu Gly Thr Pro Ser Ser Phe Ser Asn Asn Ser
    210                 215                 220

tcc gtg acg ggt gat ccg ctg atc gac gcc aat acc ggt ccc ggt gac
720
Ser Val Thr Gly Asp Pro Leu Ile Asp Ala Asn Thr Gly Pro Gly Asp
225                 230                 235                 240

agc ggc aat agc agt ggt gag gcg ggg caa ctg atc ggc gag ctt atc
768
Ser Gly Asn Ser Ser Gly Glu Ala Gly Gln Leu Ile Gly Glu Leu Ile
                245                 250                 255

gac cgt ggc ctg caa tcg gta ttg gcc ggt ggt gga ctg ggc aca ccc
816
Asp Arg Gly Leu Gln Ser Val Leu Ala Gly Gly Gly Leu Gly Thr Pro
            260                 265                 270

gta aac acc ccg cag acc ggt acg gcg gcg aat ggc gga cag tcc gct
864
Val Asn Thr Pro Gln Thr Gly Thr Ala Ala Asn Gly Gly Gln Ser Ala
        275                 280                 285

cag gat ctt gac cag ttg ctg ggc ggc ttg ctg ctc aag ggc ctt gaa
912
Gln Asp Leu Asp Gln Leu Leu Gly Gly Leu Leu Leu Lys Gly Leu Glu
    290                 295                 300

gcg acg ctc aag gat gcc ggt caa acc gct acc gac gtg cag tcg agc
960
Ala Thr Leu Lys Asp Ala Gly Gln Thr Ala Thr Asp Val Gln Ser Ser
305                 310                 315                 320

gct gcg caa atc gcc acc ttg ctg gtc agt acg ctg ctg caa ggc acc
1008
Ala Ala Gln Ile Ala Thr Leu Leu Val Ser Thr Leu Leu Gln Gly Thr
                325                 330                 335

cgc aat cag gct gca gcc tga
1029
Arg Asn Gln Ala Ala Ala
            340

<210>  29
<211>  342
<212>  PRT
<213>  Pseudomonas syringae

<400>  29
Met Gln Ser Leu Ser Leu Asn Ser Ser Ser Leu Gln Thr Pro Ala Met
1               5                   10                  15

Ala Leu Val Leu Val Arg Pro Glu Thr Glu Thr Thr Gly Ala Ser Thr
            20                  25                  30
Ser Ser Lys Ala Leu Gln Glu Val Val Val Lys Leu Ala Glu Glu Leu
        35                  40                  45
Met Arg Asn Gly Gln Leu Asp Asp Ser Ser Pro Leu Gly Lys Leu Leu
    50                  55                  60
Ala Lys Ser Met Ala Ala Asp Gly Lys Ala Gly Gly Gly Ile Glu Asp
65                  70                  75                  80
Val Ile Ala Ala Leu Asp Lys Leu Ile His Glu Lys Leu Gly Asp Asn
                85                  90                  95
Phe Gly Ala Ser Ala Asp Asn Ala Ser Gly Thr Gly Gln Gln Asp Leu
            100                 105                 110
Met Thr Gln Val Leu Ser Gly Leu Ala Lys Ser Met Leu Asp Asp Leu
        115                 120                 125
Leu Thr Lys Gln Asp Gly Gly Ala Ser Phe Ser Glu Asp Asp Met Pro
    130                 135                 140
Met Leu Asn Lys Ile Ala Gln Phe Met Asp Asp Asn Pro Ala Gln Phe
145                 150                 155                 160
Pro Lys Pro Asp Ser Gly Ser Trp Val Asn Glu Leu Lys Glu Asp Asn
                165                 170                 175
Phe Leu Asp Gly Asp Glu Thr Ala Ala Phe Arg Ser Ala Leu Asp Ile
            180                 185                 190
Ile Gly Gln Gln Leu Gly Asn Gln Gln Ser Gly Ala Gly Gly Leu Ala
        195                 200                 205
Gly Thr Gly Gly Gly Leu Gly Thr Pro Ser Ser Phe Ser Asn Asn Ser
    210                 215                 220
Ser Val Thr Gly Asp Pro Leu Ile Asp Ala Asn Thr Gly Pro Gly Asp
225                 230                 235                 240
Ser Gly Asn Ser Ser Gly Glu Ala Gly Gln Leu Ile Gly Glu Leu Ile
                245                 250                 255
Asp Arg Gly Leu Gln Ser Val Leu Ala Gly Gly Gly Leu Gly Thr Pro
            260                 265                 270
Val Asn Thr Pro Gln Thr Gly Thr Ala Ala Asn Gly Gly Gln Ser Ala
        275                 280                 285
Gln Asp Leu Asp Gln Leu Leu Gly Gly Leu Leu Leu Lys Gly Leu Glu
    290                 295                 300
Ala Thr Leu Lys Asp Ala Gly Gln Thr Ala Thr Asp Val Gln Ser Ser
305                 310                 315                 320
Ala Ala Gln Ile Ala Thr Leu Leu Val Ser Thr Leu Leu Gln Gly Thr
                325                 330                 335
Arg Asn Gln Ala Ala Ala
            340

<210>  30
<211>  1524
<212>  DNA
<213>  Artificial Sequence

<220>
<223>  DNA encoding fusion protein Harpin-TM2

<220>
<223>  Harpin (1-1026) - Linker (1027-1101) - Tamavidin2 (1102-1524)

<400>  30
atg cag agt ctc agt ctt aac agc agc tcg ctg caa acc ccg gca atg
48
Met Gln Ser Leu Ser Leu Asn Ser Ser Ser Leu Gln Thr Pro Ala Met
  1               5                  10                  15

gcc ctt gtc ctg gta cgt cct gaa acc gag acg act ggc gcc agt acg
96
Ala Leu Val Leu Val Arg Pro Glu Thr Glu Thr Thr Gly Ala Ser Thr
            20                  25                  30

tcg agc aag gcg ctt cag gaa gtt gtc gtg aag ctg gcc gag gaa ctg
144
Ser Ser Lys Ala Leu Gln Glu Val Val Val Lys Leu Ala Glu Glu Leu
        35                  40                  45

atg cgc aat ggt caa ctc gac gac agc tcg cca ttg ggc aaa ctg ctg
192
Met Arg Asn Gly Gln Leu Asp Asp Ser Ser Pro Leu Gly Lys Leu Leu
    50                  55                  60

gcc aag tcg atg gcc gcg gat ggc aag gca ggc ggc ggt atc gag gat
240
Ala Lys Ser Met Ala Ala Asp Gly Lys Ala Gly Gly Gly Ile Glu Asp
65                  70                  75                  80

gtc atc gct gcg ctg gac aag ctg att cat gaa aag ctg ggt gac aac
288
Val Ile Ala Ala Leu Asp Lys Leu Ile His Glu Lys Leu Gly Asp Asn
                85                  90                  95

ttc ggc gcg tct gcg gac aac gcc tcg ggt acc gga cag cag gac ctg
336
Phe Gly Ala Ser Ala Asp Asn Ala Ser Gly Thr Gly Gln Gln Asp Leu
            100                 105                 110

atg act cag gtg ctc agt ggc ctg gcc aag tct atg ctc gat gat ctt
384
Met Thr Gln Val Leu Ser Gly Leu Ala Lys Ser Met Leu Asp Asp Leu
        115                 120                 125

ctg acc aag cag gat ggc ggg gca agc ttc tcc gaa gac gat atg ccg
432
Leu Thr Lys Gln Asp Gly Gly Ala Ser Phe Ser Glu Asp Asp Met Pro
    130                 135                 140

atg ctg aac aag atc gcg cag ttc atg gat gac aat ccc gca cag ttt
480
Met Leu Asn Lys Ile Ala Gln Phe Met Asp Asp Asn Pro Ala Gln Phe
145                 150                 155                 160

ccc aag ccg gac tcg ggt tcc tgg gtg aac gaa ctc aag gaa gac aac
528
Pro Lys Pro Asp Ser Gly Ser Trp Val Asn Glu Leu Lys Glu Asp Asn
                165                 170                 175

ttc ctt gat ggc gac gaa acg gct gcg ttc cgc tcg gca ctc gac atc
576
Phe Leu Asp Gly Asp Glu Thr Ala Ala Phe Arg Ser Ala Leu Asp Ile
            180                 185                 190

att ggc cag caa ctg ggt aat cag cag agt ggc gct ggc ggt ctg gcg
624
Ile Gly Gln Gln Leu Gly Asn Gln Gln Ser Gly Ala Gly Gly Leu Ala
        195                 200                 205

ggg acg ggt gga ggt ctg ggc act ccg agc agt ttt tct aac aac tcg
672
Gly Thr Gly Gly Gly Leu Gly Thr Pro Ser Ser Phe Ser Asn Asn Ser
    210                 215                 220

tcc gtg acg ggt gat ccg ctg atc gac gcc aat acc ggt ccc ggt gac
720
Ser Val Thr Gly Asp Pro Leu Ile Asp Ala Asn Thr Gly Pro Gly Asp
225                 230                 235                 240

agc ggc aat agc agt ggt gag gcg ggg caa ctg atc ggc gag ctt atc
768
Ser Gly Asn Ser Ser Gly Glu Ala Gly Gln Leu Ile Gly Glu Leu Ile
                245                 250                 255

gac cgt ggc ctg caa tcg gta ttg gcc ggt ggt gga ctg ggc aca ccc
816
Asp Arg Gly Leu Gln Ser Val Leu Ala Gly Gly Gly Leu Gly Thr Pro
            260                 265                 270

gta aac acc ccg cag acc ggt acg gcg gcg aat ggc gga cag tcc gct
864
Val Asn Thr Pro Gln Thr Gly Thr Ala Ala Asn Gly Gly Gln Ser Ala
        275                 280                 285

cag gat ctt gac cag ttg ctg ggc ggc ttg ctg ctc aag ggc ctt gaa
912
Gln Asp Leu Asp Gln Leu Leu Gly Gly Leu Leu Leu Lys Gly Leu Glu
    290                 295                 300

gcg acg ctc aag gat gcc ggt caa acc gct acc gac gtg cag tcg agc
960
Ala Thr Leu Lys Asp Ala Gly Gln Thr Ala Thr Asp Val Gln Ser Ser
305                 310                 315                 320

gct gcg caa atc gcc acc ttg ctg gtc agt acg ctg ctg caa ggc acc
1008
Ala Ala Gln Ile Ala Thr Leu Leu Val Ser Thr Leu Leu Gln Gly Thr
                325                 330                 335

cgc aat cag gct gca gcc ggt ggc ggt ggc agc ggt ggc ggt ggc agc
1056
Arg Asn Gln Ala Ala Ala Gly Gly Gly Gly Ser Gly Gly Gly Gly Ser
            340                 345                 350

ggt ggc ggt ggc agc ggt ggc ggt ggc agc ggt ggc ggt ggc agc tca
1104
Gly Gly Gly Gly Ser Gly Gly Gly Gly Ser Gly Gly Gly Gly Ser Ser
        355                 360                 365

gac gtt caa tct tca ctc acc gga acc tgg tac aat gaa ctc aac tcc
1152
Asp Val Gln Ser Ser Leu Thr Gly Thr Trp Tyr Asn Glu Leu Asn Ser
    370                 375                 380

aag atg gaa ttg act gca aac aaa gac ggt act ctc act gga aag tac
1200
Lys Met Glu Leu Thr Ala Asn Lys Asp Gly Thr Leu Thr Gly Lys Tyr
385                 390                 395                 400

ctc tcc aaa gtt ggg gat gtc tac gtg ccc tac cca ctc tct ggt cgc
1248
Leu Ser Lys Val Gly Asp Val Tyr Val Pro Tyr Pro Leu Ser Gly Arg
                405                 410                 415

tat aac ctc caa ccc ccc gcg gga caa ggc gtc gct ctt ggg tgg gcg
1296
Tyr Asn Leu Gln Pro Pro Ala Gly Gln Gly Val Ala Leu Gly Trp Ala
            420                 425                 430

gta tcc tgg gag aac agt aaa att cat tcc gct acg aca tgg agc gga
1344
Val Ser Trp Glu Asn Ser Lys Ile His Ser Ala Thr Thr Trp Ser Gly
        435                 440                 445

cag ttc ttc tct gag tcg tct cca gtg att ctt act cag tgg ttg ttg
1392
Gln Phe Phe Ser Glu Ser Ser Pro Val Ile Leu Thr Gln Trp Leu Leu
    450                 455                 460

tca tcg agc act gcg cgt ggg gac gta tgg gaa tcc aca ctt gtg ggg
1440
Ser Ser Ser Thr Ala Arg Gly Asp Val Trp Glu Ser Thr Leu Val Gly
465                 470                 475                 480

aat gat tcg ttt aca aag acg gcg ccg act gag cag cag atc gct cat
1488
Asn Asp Ser Phe Thr Lys Thr Ala Pro Thr Glu Gln Gln Ile Ala His
                485                 490                 495

gct caa ctc cat tgt cgc gca ccg agg ttg aag taa
1524
Ala Gln Leu His Cys Arg Ala Pro Arg Leu Lys
            500                 505

<210>  31
<211>  507
<212>  PRT
<213>  Artificial Sequence

<220>
<223>  Fusion protein Harpin-TM2

<220>
<223>  Harpin (1-342) - Linker (343-367) - Tamavidin2 (368-507)

<400>  31
Met Gln Ser Leu Ser Leu Asn Ser Ser Ser Leu Gln Thr Pro Ala Met
  1               5                  10                  15
Ala Leu Val Leu Val Arg Pro Glu Thr Glu Thr Thr Gly Ala Ser Thr
             20                  25                  30
Ser Ser Lys Ala Leu Gln Glu Val Val Val Lys Leu Ala Glu Glu Leu
         35                  40                  45
Met Arg Asn Gly Gln Leu Asp Asp Ser Ser Pro Leu Gly Lys Leu Leu
     50                  55                  60
Ala Lys Ser Met Ala Ala Asp Gly Lys Ala Gly Gly Gly Ile Glu Asp
 65                  70                  75                  80
Val Ile Ala Ala Leu Asp Lys Leu Ile His Glu Lys Leu Gly Asp Asn
                 85                  90                  95
Phe Gly Ala Ser Ala Asp Asn Ala Ser Gly Thr Gly Gln Gln Asp Leu

                100                 105                 110
Met Thr Gln Val Leu Ser Gly Leu Ala Lys Ser Met Leu Asp Asp Leu
        115                 120                 125
Leu Thr Lys Gln Asp Gly Gly Ala Ser Phe Ser Glu Asp Asp Met Pro
    130                 135                 140
Met Leu Asn Lys Ile Ala Gln Phe Met Asp Asp Asn Pro Ala Gln Phe
145                 150                 155                 160
Pro Lys Pro Asp Ser Gly Ser Trp Val Asn Glu Leu Lys Glu Asp Asn
                165                 170                 175
Phe Leu Asp Gly Asp Glu Thr Ala Ala Phe Arg Ser Ala Leu Asp Ile
            180                 185                 190
Ile Gly Gln Gln Leu Gly Asn Gln Gln Ser Gly Ala Gly Gly Leu Ala
        195                 200                 205
Gly Thr Gly Gly Gly Leu Gly Thr Pro Ser Ser Phe Ser Asn Asn Ser
    210                 215                 220
Ser Val Thr Gly Asp Pro Leu Ile Asp Ala Asn Thr Gly Pro Gly Asp
225                 230                 235                 240
Ser Gly Asn Ser Ser Gly Glu Ala Gly Gln Leu Ile Gly Glu Leu Ile
                245                 250                 255
Asp Arg Gly Leu Gln Ser Val Leu Ala Gly Gly Gly Leu Gly Thr Pro
            260                 265                 270
Val Asn Thr Pro Gln Thr Gly Thr Ala Ala Asn Gly Gly Gln Ser Ala
        275                 280                 285
Gln Asp Leu Asp Gln Leu Leu Gly Gly Leu Leu Leu Lys Gly Leu Glu
    290                 295                 300
Ala Thr Leu Lys Asp Ala Gly Gln Thr Ala Thr Asp Val Gln Ser Ser
305                 310                 315                 320
Ala Ala Gln Ile Ala Thr Leu Leu Val Ser Thr Leu Leu Gln Gly Thr
                325                 330                 335
Arg Asn Gln Ala Ala Ala Gly Gly Gly Gly Ser Gly Gly Gly Gly Ser
            340                 345                 350
Gly Gly Gly Gly Ser Gly Gly Gly Gly Ser Gly Gly Gly Gly Ser Ser
        355                 360                 365
Asp Val Gln Ser Ser Leu Thr Gly Thr Trp Tyr Asn Glu Leu Asn Ser
    370                 375                 380
Lys Met Glu Leu Thr Ala Asn Lys Asp Gly Thr Leu Thr Gly Lys Tyr
385                 390                 395                 400
Leu Ser Lys Val Gly Asp Val Tyr Val Pro Tyr Pro Leu Ser Gly Arg
                405                 410                 415
Tyr Asn Leu Gln Pro Pro Ala Gly Gln Gly Val Ala Leu Gly Trp Ala
            420                 425                 430
Val Ser Trp Glu Asn Ser Lys Ile His Ser Ala Thr Thr Trp Ser Gly
        435                 440                 445
Gln Phe Phe Ser Glu Ser Ser Pro Val Ile Leu Thr Gln Trp Leu Leu
    450                 455                 460
Ser Ser Ser Thr Ala Arg Gly Asp Val Trp Glu Ser Thr Leu Val Gly
465                 470                 475                 480
Asn Asp Ser Phe Thr Lys Thr Ala Pro Thr Glu Gln Gln Ile Ala His
                485                 490                 495
Ala Gln Leu His Cys Arg Ala Pro Arg Leu Lys
            500                 505

## Description

### TECHNICAL FIELD

The present invention relates to a method for qualitatively and/or quantitatively detecting a substance in a biological sample. In particular, the method of the present invention can detect even a trace amount of substance that is present in a biological sample and that cannot be readily detected in usual manner.

### BACKGROUND ART

### Method for detecting a substance to be detected utilizing a carrier on which a protein that can specifically binds to the substance is immobilized by hydrophobic bonding, covalent bonding, etc.

In order to detect a substance in a biological sample, a method utilizing a substance that specifically binds to the former substance (substance to be detected) has been widely used. As the substances that specifically bind to the substance to be detected, for example, antibodies or other proteins are usually used. These substances can be immobilized on a carrier such as a microplate, microbeads, or a sensor chip. For example, hydrophobic bonding and covalent bonding are known as general means for the immobilization.

In the "hydrophobic bonding", a carrier and a protein that specifically binds to a substance to be detected (hereinafter, may be referred to as "specific protein") are bound to each other by interaction between the hydrophobic surface of the carrier and the hydrophobic moiety of the specific protein. This is convenient from the point of not needing specific reagents. However, such binding is usually weak. In the case where the hydrophobic bonding is applied to, for example, enzyme-linked immunosorbent assay (ELISA), the protein is detached from the carrier during, for example, a washing procedure after binding in many cases. Furthermore, in the case where a specific protein is bound to a carrier by hydrophobic bonding, the function of the protein may be lost completely or partially in many cases.

The "covalent bonding" utilizes interaction between functional groups (e.g., amino groups) of a specific protein and functional groups (e.g., carboxyl group) provided on the surface of the carrier, and is strong. However, after a specific protein is bound to a carrier by covalent bonding, the function of the protein is lost completely or partially in many cases, like the hydrophobic bonding.

In addition to the hydrophobic bonding and the covalent bonding, known is a method for fusing a plurality of histidine molecules to terminals of protein molecules and binding the fusion protein having the histidine tags to, for example, a base plate, such as a protein chip, having a surface provided with nickel. The interaction between the histidine tags and nickel ions is, however, not very strong, and nickel ions are known to non-specifically bind to a variety of biological molecules.

In such a specific binding assay system using a solid phase on which a protein that specifically binds to a substance to be detected is bound by hydrophobic bonding or covalent bonding, non-specific binding, which causes background signals and thus should be reduced, is generally a severe problem. In order to solve this problem, the following methods have been proposed for example: a method of adding an extract of a bacterium component to a reagent for detection (JP No. S59-99257 A (1984)); a method of adding a culture component of host cells containing a vector of the same species as that used in production of a recombinant protein capable of specifically binding to a substance to be detected and the vector not containing the gene encoding the protein to a sample (JP No. H8-43392 A (1996)); and a method of heat-treating an aqueous extract from cells of the same species as that producing a recombinant protein capable of specifically binding to a substance to be detected, and the cell not containing this protein, and then adding water-soluble fraction of the heated aqueous extract to a sample (JP No.2004-301646 A). These methods show some effects on inhibition of non-specific binding.

### Detection of substance utilizing avidin-biotin binding

Avidin is a glycoprotein derived from egg white and extremely strongly binds to biotin (vitamin H). The interaction between avidin and biotin is one of the strongest noncovalent bonds (Green, (1975), Adv Protein Chem, 29: 85-133). On the other hand, streptavidin is an avidin-like protein derived from *Streptomyces avidinii* and also strongly binds to biotin. The interaction of (strept)avidin-biotin, because of its high acting force, has been widely applied, for example, for detection of antigens and antibodies in the fields of molecular biology and biochemistry (Green, (1990), Methods Enzymol, 184: 51-67).

A method has been proposed for binding a protein to a carrier by the biotin-binding ability of avidin or streptavidin. That is, the method involves binding (strept)avidin to a base plate such as a microplate by covalent bonding or hydrophobic bonding and further binding to a biotinylated protein to immobilize the protein.

A technology of immobilizing base plate-biotin-avidin-biotin-desired protein in this order is also reported in which an avidin protein is bound to a base plate provided with biotin by avidin-biotin binding and then a biotinylated desired protein thereto at another biotin pocket of the avidin (JP No. H4-236353 A (1992)). A substance to be detected can be detected using a plate on which a specific protein is immobilized by such a method.

The assay utilizing the avidin-biotin binding has also a big problem with a large background signal, like the assay using a solid phase on which a protein that specifically binds to a substance to be detected is bound by, for example, hydrophobic bonding or covalent bonding. Countermeasures have been proposed for solving this problem are, for example: a method in which a sample is put into contact with a solid phase to which inactivated (strept)avidin is bound and then contact with a solid phase to which active (strept)avidin is bound (JP No. H8-114590 A (1996)); a method in which a biotinylated substance is bound to a avidin-bound solid phase, and then this is put into contact with a conjugate of polyethylene glycol and biotin (JP No. H11-211727 A (1999)); and a method in which a biotin-containing solution is put into contact with a solid phase (JP No. 2002-48794 A), in addition to the above-mentioned methods for preventing non-specific binding. Unfortunately, all the methods exhibit insufficient practical advantages.

In the past, fusion proteins have been produced using avidin or streptavidin in order to label a protein or to use as a diagnostic marker or a targeted cell-specific factor (Airenne et al., (1999), Biomol Eng, 16: 87-92). Application of these fusion proteins, in particular, fusion proteins of avidin or streptavidin and antibodies such as scFv, Fab fragment, or IgG to agents for specifically targeting, for example, cancer cells has been studied. In addition, an idea of a column is described on which scFv is immobilized through avidin-biotin binding using a fusion protein between streptavidin and scFv (Kiprivanov et al., (1995), Hum Antib Hybrid, 6: 93-101; Dubel et al., (1995), J Immunol Methods, 178: 201-209). However, no example is known on immobilization using a biotin-binding protein to detect a substance to be detected in a biological sample. There is a report on an example in which ELISA is performed by immobilizing a streptavidin fusion protein on a biotinylated plate and using an antibody for the fusion protein as the antigen (WO2002/046395), but this merely demonstrates that a streptavidin fusion protein can be immobilized on a carrier without losing its activity.

### CITATION LIST

### PATENT DOCUMENT

Patent Document 1: JP No. S59-99257 A
Patent Document 2: JP No. H8-43392 A
Patent Document 3: JP No. 2004-301646 A
Patent Document 4: JP No. H8-114590 A
Patent Document 5: JP No. H11-211727 A
Patent Document 6: JP No. 2002-48794 A
Patent Document 7: WO2002/046395
Patent Document 8: WO2002/072817

### NON-PATENT DOCUMENT

Non-Patent Document 1: Green, (1975), Adv Protein Chem, 29: 85-133
Non-Patent Document 2: Green, (1990), Methods Enzymol, 184: 51-67
Non-Patent Document 3: Airenne et al., (1999), Biomol Eng, 16: 87-92
Non-Patent Document 4: Kiprivanov et al., (1995), Hum Antib Hybrid, 6: 93-101
Non-Patent Document 5: Dubel et al., (1995), J Immunol Methods, 178: 201-209
Non-Patent Document 6: Takakura et al., (2009), FEBS J, 276: 1383-1397

### SUMMARY OF INVENTION

### TECHNICAL PROBLEM

It is an object of the present invention to provide a method for qualitatively and/or quantitatively detecting a substance in a biological sample.

Specifically, it is an object of the present invention to provide a method for qualitatively and/or quantitatively detecting a trace amount of substance that is present in a biological sample while reducing the background signal level.

### SOLUTION TO PROBLEM

The inventors have diligently studied and, as a result, have developed a system in which a fusion protein between a protein that specifically binds to a substance to be detected and a biotin-binding protein is immobilized on a carrier through binding between biotin and the biotin-binding protein. Furthermore, the inventors have discovered that, in particular, in order to detect a trace amount of substance that is present in a biological sample in this system, a countermeasure against the background signal is very important and have arrived at the present invention through a measure for reducing the background signal level. Specifically, a reduction in nonspecific binding was significant after a cell homogenate extract and a biotin-binding protein were added to a biological sample. Alternatively, addition of a cell homogenate extract prepared from cells genetically engineered to bind a biotin-binding protein, instead of the biotin-binding protein, achieved substantially the same effect.

In addition, the present inventors have discovered that, in the system in which a fusion protein between a protein that specifically binds to a substance to be detected and a biotin-binding protein is immobilized on a carrier, a lower concentration of substance can be stably measured, with a reduced background signal level, by putting (blocking) the biotin-binding protein into contact with the carrier before the addition of a biological sample.

Based on the knowledge described above, the present invention provides a method of high-sensitive detection with reduced non-specific binding in a system in which a substance that specifically detects a substance to be detected is immobilized on a carrier through avidin-biotin binding.

The present invention includes the following nonlimiting embodiments.

### [Embodiment 1]

A method for detecting a substance in a biological sample, which comprises:
1) providing a carrier on which biotin is bound and providing a fusion protein between a protein that specifically binds to a substance to be detected and a biotin-binding protein;
2) binding the fusion protein to the carrier provided in step 1) through binding between biotin and the biotin-binding protein to produce a fusion protein-bound carrier;
3) mixing
   (a) a biological sample, and
   (b-i) a cell homogenate extract prepared from cells of the same species as the host cells used for expressing the fusion protein in step 1), and a biotin-binding protein, or
   (b-ii) a cell homogenate extract prepared from cells of the same species as the host cells used for expressing the fusion protein in step 1), and genetically engineered to express a biotin-binding protein,
   and adding the mixture to the fusion protein-bound carrier produced in step 2); and
4) detecting a substance that has bound to the protein that specifically binds to a substance to be detected in the fusion protein.

### [Embodiment 2]

A method for detecting a substance in a biological sample, which comprises:
1) providing a carrier on which biotin is bound and providing a fusion protein between a protein that specifically binds to a substance to be detected and a biotin-binding protein;
2) binding the fusion protein to the carrier provided in step 1) through binding between biotin and the biotin-binding protein to produce a fusion protein-bound carrier;
3) putting a biotin-binding protein into contact with the fusion protein-bound carrier produced in step 2) to block the carrier;
4) after the blocking step in step 3), adding a biological sample to the fusion protein-bound carrier; and
5) detecting a substance to be detected that has bound to the protein that specifically binds to the substance in the fusion protein.

### [Embodiment 3]

A method for detecting a substance in a biological sample, which comprises:
1) providing a carrier on which biotin is bound and providing a fusion protein between a protein that specifically binds to a substance to be detected and a biotin-binding protein;
2) binding the fusion protein to the carrier provided in step 1) through binding between biotin and the biotin-binding protein to produce a fusion protein-bound carrier;
3) putting a biotin-binding protein into contact with the fusion protein-bound carrier produced in step 2) to block the carrier;
4) after the blocking step in step 3), mixing
   (a) a biological sample, and
   (b-i) a cell homogenate extract prepared from cells of the same species as the host cells used for expressing the fusion protein in step 1), and a biotin-binding protein, or
   (b-ii) a cell homogenate extract prepared from cells of the same species as the host cells used for expressing the fusion protein in step 1), and genetically engineered to express a biotin-binding protein,
   and adding the mixture to the fusion protein-bound carrier; and
5) detecting a substance to be detected that has bound to the protein that specifically binds to the substance in the fusion protein.

### [Embodiment 4]

The method according to Embodiment 1 or 3, wherein step 3(b-i) in Embodiment 1 or step 4(b-i) in Embodiment 3 comprises adding a cell homogenate extract extracted from cells comprising any vector, as the cell homogenate extract.

### [Embodiment 5]

The method according to any one of Embodiments 1 to 4, wherein the biotin-binding protein is tamavidin or a variant thereof.

### [Embodiment 6]

The method according to any one of Embodiments 1 to 5, wherein the biological sample is selected from the group consisting of blood, serum, cerebrospinal fluid, saliva, sweat, urine, tear, lymph fluid, and mother's milk.

### [Embodiment 7]

A carrier for detecting a substance in a biological sample, wherein
a fusion protein between a protein that specifically binds to a substance to be detected and a biotin-binding protein is bound to a carrier by binding between biotin and the biotin-binding protein, wherein
the fusion protein is bound to the carrier by a method comprising:
1) providing a carrier on which biotin is bound and providing a fusion protein between a protein that specifically binds to a substance to be detected and a biotin-binding protein;
2) binding the fusion protein to the carrier provided in step 1) through binding between biotin and the biotin-binding protein to produce a fusion protein-bound carrier; and
3) putting a biotin-binding protein into contact with the fusion protein-bound carrier produced in step 2) to block the carrier.

### [Embodiment 8]

A kit for detecting a substance in a biological sample, which comprises:
A) a carrier on which a fusion protein between a protein that specifically binds to a substance to be detected and a biotin-binding protein is bound by binding between biotin and the biotin-binding protein; and
   an agent for diluting a biological sample, comprising
   B-i) a cell homogenate extract prepared from cells of the same species as the host cells used for expressing the fusion protein in step A), and a biotin-binding protein, or
   B-ii) a cell homogenate extract prepared from cells of the same species as the host cells used for expressing the fusion protein in step A), and genetically engineered to express a biotin-binding protein; and/or
C) a blocking agent containing a biotin-binding protein.

### ADVANTAGEOUS EFFECTS OF INVENTION

The method of the present invention enables high-sensitivity and stable detection, with a reduced background signal level, of a substance to be detected in a biological sample. In particular, the method of the present invention enables detection of a trace amount of substance which is present in a biological sample and, usually, cannot be readily detected.

### BRIEF DESCRIPTION OF THE DRAWINGS

Fig. 1 includes schematic diagrams illustrating blocking by a biotin-binding protein. The open circles represent biotin, the closed ellipses represent a biotin-binding protein, and the open ellipses represent a protein that specifically binds to a substance to be detected:
   Fig. 1A: without blocking by a biotin-binding protein and with immobilization of a fusion protein; Fig. 1B: without blocking by a biotin-binding protein and without immobilization of a fusion protein; Fig. 1C: with blocking by a biotin-binding protein and with immobilization of a fusion protein; and Fig. 1D: with blocking by a biotin-binding protein and without immobilization of a fusion protein.
Fig. 2 illustrates detection by Western blotting of a fusion protein between SITH-1 and TM2 expressed in E. coli. E. coli containing a vector alone was used as a control.

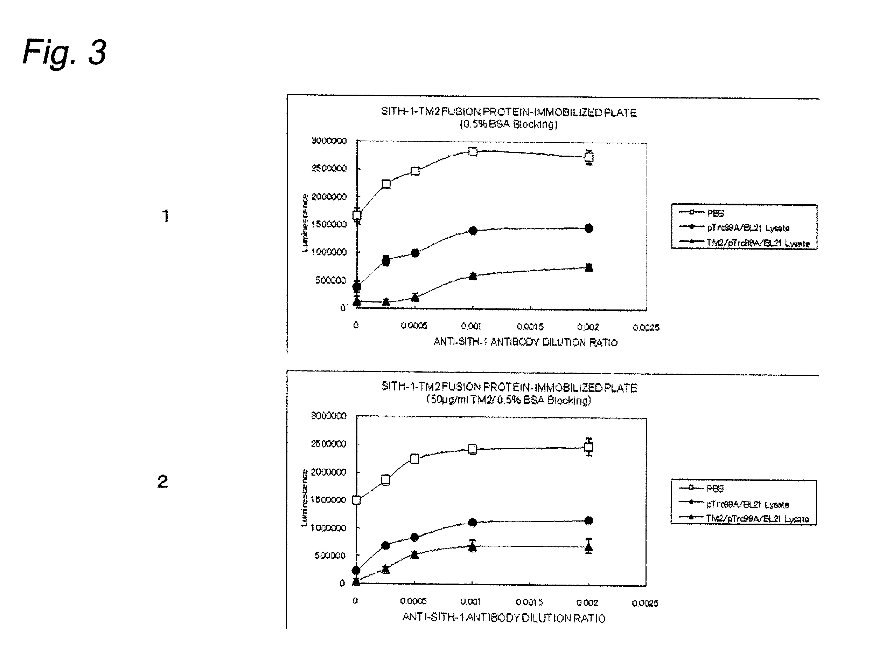

Fig. 3 shows the effect of the addition of an E. coli homogenate extract to serum on non-specific binding and the blocking effect of tamavidin 2. Fig. 3-1 shows the result of blocking by BSA (only), that is, the result obtained by subtracting the measured value in a biotinylated plate (0.5% BSA Blocking) on which nothing is immobilized from the measured value in a SITH-1-TM2 fusion protein-immobilized plate (0.5% BSA Blocking). Fig. 3-2 shows the result of blocking by BSA and TM2, that is, the result obtained by subtracting the measured value in a biotinylated plate (50 µg/mL TM2/0.5% BSABlocking) on which nothing is immobilized from the measurement value in a SITH-1-TM2 fusion protein-immobilized plate (50 µg/mL TM2/0.5% BSABlocking).
Fig. 4 illustrates detection of a fusion protein between Harpin and TM2 expressed in E. coli in lane 2 by Western blotting. E. coli containing a vector alone was used (lane 1) as a control.

### EMBODIMENTS OF INVENTION

### I. Method of detection of the present invention (Embodiment 1)

The method of detection (Embodiment 1) according to the present invention relates to a method for detecting a substance in a biological sample and comprises:
1) providing a carrier on which biotin is bound and providing a fusion protein between a protein that specifically binds to a substance to be detected and a biotin-binding protein;
2) binding the fusion protein to the carrier provided in step 1) through binding between biotin and the biotin-binding protein to produce a fusion protein-bound carrier;
3) mixing
   (a) a biological sample, and
   (b-i) a cell homogenate extract prepared from cells of the same species as the host cells used for expressing the fusion protein in step 1), and a biotin-binding protein, or
   (b-ii) a cell homogenate extract prepared from cells of the same species as the host cells used for expressing the fusion protein in step 1), and genetically engineered to express a biotin-binding protein,
   and adding the mixture to the fusion protein-bound carrier produced in step 2); and
4) detecting a substance that has bound to the protein that specifically binds to a substance to be detected in the fusion protein.

### Substance to be detected in biological sample

The present invention relates to a method for detecting a substance in a biological sample.

Any biological sample that is expected to contain a substance as a detection target can be used in the present invention without limitation. Examples of the sample include cells and tissues collected from organisms and fragments thereof, for example, humors, more preferably, blood, serum, cerebrospinal fluid, saliva, sweat, urine, tear, lymph fluid, and mother's milk.

These humors may be used after dilution as needed. The dilution rate is, but not limited to, generally in the range of about 2 to about 10000 fold, preferably about 100 to 1000 fold. The diluent may be any buffer solution, which may contain any proper blocking agent. Preferred blocking agents have high inhibitory effect on nonspecific binding, and can be selected from blocking agents well-known to persons skilled in the art, such as BSA and casein. Note that in Embodiment 2 of the present invention, the blocking agent is a biotin-binding protein. This will be described below.

The substance to be detected in the present invention is any substance that is desired to be detected or measured in a biological sample, and preferred examples thereof include proteins such as antibodies and antigens and their fragments, peptides, nucleic acids, carbohydrates, and glycolipids.

The present invention enables measurement of a trace amount of substance that is present in a biological sample and cannot be readily detected or accurately determined quantitatively by conventional methods. For example, if the substance to be detected is an antibody exhibiting a low antibody titer in a serum (e.g., a low antibody titer not detectable at 1000-fold dilution, but detectable at 100-fold dilution with difficulty), a low dilution rate of the serum is required. As a result, non-specific binding derived from serum components inevitably increases. Thus, no known method enables detection or determination of quantity for the antibody. In contrast, the method of the present invention can readily detect and accurately determine quantity of the antibody.

Nonlimiting examples of the substance to be detected in the present invention include antibodies against a small protein encoded by the intermediate transcript of HHV-6 (SITH-1). Other examples include antibodies against other antigens of herpes viruses, antibodies against virus-related antigens derived from, for example, cytomegaloviruses, hepatitis viruses, HIVs, HTLVs, measles viruses, and influenza viruses, antibodies against bacterium-related antigens derived from, for example, Helicobacter pylori, and antibodies against fungi-related antigens.

### SITH-1 based on Description in PCT/JP2008/67300 and U.S. Provisional Application No. 61/102441

### (1) SITH-1 Protein and Nucleic Acid

The structures and functions of the SITH-1 protein and a nucleic acid are disclosed in PCT/JP2008/67300, and the entity thereof is incorporated therein.

The SITH-1 is a factor involving latent infection with herpes viruses, and more particularly, a protein specifically expressed during latent infection with herpes viruses. The term "specifically expressed during latent infection with herpes viruses" therein refers to specific expression of genes or gene products derived from herpes viruses during latent infection (not productive infection) with herpes viruses in hosts infected with herpes viruses.

Examples of the SITH-1 protein and the nucleic acid include (a) a protein which has an amino acid sequence of SEQ ID NO: 1 and a nucleic acid encoding the protein.

The SITH-1 protein having the amino acid sequence of SEQ ID NO: 1, as described in Reference Example below, was isolated and identified as a protein that is specifically expressed during latent infection with human herpes viruses 6 (HHV-6). The SITH-1 protein is a protein having the amino acid sequence of SEQ ID NO: 1, composed of 159 amino acids, and having a molecular mass of about 17.5 kDa.

The SITH-1 protein is encoded by the nucleic acid of the SITH-1 gene. The cDNA of this SITH-1 gene, as shown in SEQ ID NO: 3, has a size of 1795 base pairs (about 1.79 kbp), the nucleotide sequence from the 954th to 956th being the initiation codon (Kozak ATG), while the nucleotide sequence from 1431st to 1433rd being the termination codon (TAA). Accordingly, the SITH-1 nucleic acid has a nucleotide sequence from 954th to 1430th as an open reading frame (ORF) in the nucleotide sequence of SEQ ID NO: 3, the ORF having a size of 477 base pairs (about 0.48 kbp). In the cDNA of the SITH-1, the nucleotide sequence representing the ORF region is shown in SEQ ID NO: 2. The nucleotide sequence of SEQ ID NO: 2 includes three bases of the stop codon.

The SITH-1 nucleic acid is always expressed in the cytoplasm of a cell latent-infected with HHV-6, but not in a productively infected cell. The nucleic acid encoding the SITH-1 protein is encoded by a DNA that is a complementary strand of the HHV-6 latent infection specific gene (H6LT), which has been reported to date, and its expression is enhanced in the intermediate stage of the latent infection with HHV-6. These facts demonstrate that the SITH-1 protein is a protein that is specifically expressed during latent infection with HHV-6.

The SITH-1 protein binds to a host protein, CAML (calcium-modulating cyclophilin ligand, Accesion #: U18242) to increase the calcium concentration in the glial cells. The CAML is a protein that is known to be abundantly present in the brain and lymphocytes in the host living organism and increase the intracellular calcium concentration. It is considered that an increase in intracellular calcium concentration due to expression of the SITH-1 protein probably leads to activation of overall signaling in the latent-infected cells, and thus contributes to efficient reactivation of HHV-6.

It is known that the glial cells in the brain are latent-infected with HHV-6. When HHV-6 during the latent infection or at the intermediate stage which is a latent infection state with high activity expresses the SITH-1, the calcium concentration seems to increase in the glial cells. It is believed that an increase in intracellular calcium concentration in the brain is wedded to psychiatric disorders such as mood disorders (Riken Annual Report 2003).

The SITH-1 protein has a function that maintains activity to bind to the host protein, CAML to increase the intracellular calcium concentration. Furthermore, expression of the SITH-1 protein in the glial cells, in which this protein seems to be most strongly expressed, in the brain can induce psychiatric disorders. Accordingly, the SITH-1 protein is believed to be expressed during the latent infection with herpes viruses or at the initial stage of reactivation of the herpes viruses to cause the host to have any psychiatric disorder.

### (2) Antibody against SITH-1

The antibody against the SITH-1 can be prepared as a polyclonal antibody or a monoclonal antibody from the SITH-1 protein, its variant, or their partial peptides as antigen by a known process. Examples of the known process are described in documents such as Harlow et al., "Antibodies: A laboratory manual (Cold Spring Harbor Laboratory, New York (1988))" and Iwasaki et al., "Monoclonal Antibody: Hybridoma and ELISA, Kodansha (1991)". The resulting antibody can be used for detection and determination of the SITH-1 protein.

The term "antibody" refers to immunoglobulins (IgA, IgD, IgE, IgG, IgM, and Fab fragments, F(ab')₂ fragments, and Fc fragments thereof). Examples of the antibody include, but not limited to, polyclonal antibodies, monoclonal antibodies, single-stranded antibodies, antiidiotype antibodies, and humanized antibodies.

The term "antibody recognizing the SITH-1 protein" includes complete molecules and antibody fragments specifically attachable to the SITH-1 protein (for example, Fab and F(ab')₂ fragments). Fab, F(ab')₂, and other fragments of the SITH-1 antibody can be used according to the method disclosed in the present specification or any known method. Such fragments can be typically produced by cleavage by proteolysis using an enzyme, e.g., papain (yielding a Fab fragment) or pepsin (yielding an F(ab')₂ fragment).

It is believed that patients having mood disorders and individuals having potential mood disorders exhibit increased expression levels of the SITH-1 protein and thus increased SITH-1 antibody titers. In one embodiment of the present invention, detection of the SITH-1 antibody in a biological sample enables identification of patients having mood disorders and individuals having potential mood disorders.

### Carrier on which fusion protein between protein specifically binding to substance to be detected and biotin-binding protein is bound (steps 1) and 2) of Example 1)

The method of detection according to the present invention uses a carrier on which a fusion protein between a protein that specifically binds to a substance to be detected and a biotin-binding protein is bound by binding between biotin and the biotin-binding protein.

### The carrier of the present invention can be produced by a method including:

1) providing a carrier on which biotin is bound and providing a fusion protein between a protein that specifically binds to a substance to be detected and a biotin-binding protein; and
2) binding the fusion protein to the carrier provided in step 1) through binding between biotin and the biotin-binding protein to produce a fusion protein-bound carrier.

### Biotin-bound carrier

"Biotin" is a generic name of D-[(+)-cis-hexahydro-2-oxo-1H-thieno-(3,4)-imidazole-4-valeric acid]. It is one of water-soluble vitamin categorized into a vitamin B group, and is also referred to as vitamin B₇, vitamin H, or coenzyme R. Biotin very strongly binds to avidin, one of the glycoproteins contained in egg white, so that its absorption is precluded. Thus, large dose of uncooked egg white may cause biotin deficiency disease in some cases.

The term "biotin" throughout the specification includes iminobiotin (Hofmann et al., (1980), Proc Natl Acad Sci USA, 77: 4666-4668), desthiobiotin (Hirsch et al., (2002), Anal Biochem, 308: 343-357), and biotin analogs such as biocytin and biotin sulfoxide, in addition to the biotin described above.

Systems using avidin (biotin-binding protein)/biotin complexes are widely used in the fields of tissue immunology, DNA analysis, and clinical assay. In the method for binding a protein to a carrier according to the present invention, a fusion protein composed of a biotin-binding protein and a desired protein is bound to a carrier by avidin-biotin binding. The method of the present invention ensures more efficient action of the protein, without impairing the function thereof, than conventional binding by avidin-biotin binding.

Examples of materials for the solid carrier include, but not limited to, cellulose, Teflon (registered trademark), nitrocellulose, agarose, dextran, chitosan, polystyrene, polyacrylamide, polyesters, polycarbonates, polyamides, polypropylene, nylons, polydivinylidene difluoride, latex, silica, glass, glass fiber, gold, platinum, silver, copper, iron, stainless steel, ferrite, silicon wafer, polyethylene, polyethyleneimine, poly(lactic acid), resin, polysaccharides, proteins such as albumin, carbon, and combination thereof. Preferred materials have a certain level of strength, a stable composition, and reduced non-specific binding.

Examples of the form of the solid carrier include, but not limited to, beads, magnetic beads, thin films, microtubes, filters, plates, microplates, carbon nanotubes, and sensor chips. Flat solid carriers such as thin films and plates may be provided with pits, grooves, or filter bottoms, as is known in the art.

In an embodiment of the invention, beads may have a spherical diameter in the range of about 25 nm to about 1 mm. In a preferred embodiment, the beads may have a diameter in the range of about 50 nm to about 10 µm. The size of the beads may be selected depending on the specific application. Since some bacterial spores have a size of an order of about 1 µm, preferred beads for capturing such spores have a diameter larger than 1 µm.

Without any limitation, for example, in the case that high sensitivity for detection is desired, beads as described above can preferably be used as the solid carrier in the view point that the beads provide high contacting frequency between the target substance to be detected and the substance that specifically binds to the target substance, and that cleaning operation thereof is easy.

An exemplary method of binding biotin to the carrier involves use of a biotinylation reagent. Examples of the biotinylation reagent include, but not limited to, EZ-Link (registered trademark) Sulfo-NHS-Biotin (the length of the linker: 13.5 angstroms, the reactive group: primary amine, hereinafter the same order), EZ-Link (registered trademark) Sulfo-NHS-LC-Biotin (22.4 angstroms, primary amine), EZ-Link (registered trademark) Sulfo-NHS-LCLC-Biotin (30.5 angstroms, primary amine), EZ-Link (registered trademark) PFP-Biotin (9.6 angstroms, amine), EZ-Link (registered trademark) Maleimide-PEO₂-Biotin (29.1 angstroms, thiol group), EZ-Link (registered trademark) Biotin-PEO₂ Amine (20.4 angstroms, carboxyl group), EZ-Link (registered trademark) Biotin-PEO₃-LC Amine (22.9 angstroms, carboxyl group), EZ-Link (registered trademark) Biotin-Hydrazide (15.7 angstroms, aldehyde group), EZ-Link (registered trademark) Biotin-LC-Hydrazide (24.7 angstroms, aldehyde group), and EZ-Link (registered trademark) NHS-Iminobiotin (13.5 angstroms, primary amine), which are available from PIERCE.

Using these biotinylation reagent, biotin can be bound to a desired carrier such a microplate, microbeads, or a sensor chip by any known process. For example, various carriers having functional groups, such as amino, carboxyl, thiol, tosyl, epoxy, and maleimide groups, and activated ester (for example, magnetic beads, Sepharose beads, agarose beads, latex beads, and microtiter plates) can be used. For example, in the case of the use of a biotinylation reagent containing NHS ester, the reagent may be dissolved in an organic solvent such as dimethyl sulfoxide (DMSO) or phosphate buffer of pH 7 to 9, and then may be added to an immobilization carrier having amino groups to bind biotin thereto. In the case of the use of a biotinylation reagent containing amino groups, the carboxyl groups on the immobilization carrier may be converted to activated ester using carbodiimide such as 1-ethyl-3-(3-dimethylaminopropyl) carbodiimide hydrochloride (EDC), followed by addition of a biotinylation reagent dissolved in buffer solution (pH: about 5) to bind biotin to the carrier. The biotinylated immobilization carrier is preferably blocked with BSA after inactivation of unreacted functional groups.

Commercially available biotinylated carriers can also be used. Examples of the biotinylated microplates include, but not limited to, Reacti-Bind^{™} Biotin Coated Polystyrene Plates (PIERCE). Examples of the biotinylated microbeads include, but not limited to, magnetic beads, such as BioMag Biotin (available from Polysciences), magnetic nanobeads, such as nanomag (registered trademark)-D biotin and nanomag (registered trademark)-silica biotin available from Corefront, polystyrene microbeads, such as Beadlyte (registered trademark) Biotin Beads (available from Upstate), agarose, such as Biotin Agarose and 2-iminobiotin-Agarose available from Sigma, and highly cross-linked agarose, such as Biotin-Sepharose (available from Biosearch Technologies, Inc.).

The length of the linker binding the carrier to biotin is preferably at least 5 angstroms, more preferably at least 13.5 angstroms, more preferably at least 22.4 angstroms, and most preferably at least 30.5 angstroms.

### Protein specifically binds to substance to be detected

The protein that specifically binds to a substance to be detected (hereinafter may be referred to as "specific protein") is not particularly limited In one embodiment of the present invention, for example, one of the antigen and antibody, ligand such as hormone and receptor, lectin and saccharide, or complementary bindings of nucleic acid in a sample to be tested is selectively analyzed by means of the ability to form a specific complex with the other, but the specific protein is not limited thereto.

More specifically, examples of the protein include, but not limited to, antibodies, antigenic proteins, lectins, peptides, protein A, protein G, protein L, receptors, and enzymatic proteins. Examples of the antibody include antibody fragments containing antigen-binding sites, such as scFv and Fab, in addition to IgG. Examples of the antigenic protein include proteins derived from viruses such as hepatitis B and C viruses, HIVs, influenza viruses, and herpes viruses; proteins derived from bacteria such as Helicobacter pylori; tumor markers such as CEA and PSA; and sex hormones. The lectin is a saccharide-binding protein, and examples thereof include monosaccharide specific lectins such as mannose specific lectin, GalNAc specific lectin, GlcNAc specific lectin, fucose specific lectin, and sialic acid specific lectin, and oligosaccharide specific lectins. Furthermore, the examples include DNA/RNA binding proteins. Examples of the peptide include those composed of 2 to 100 amino acids, preferably 4 to 50 amino acids, and more preferably 6 to 30 amino acids.

Furthermore, in the present invention, examples of the protein that specifically binds to a substance to be detected include, but not limited to, SITH-1 proteins.

### Biotin-binding protein

The present invention includes a method of immobilizing a protein that specifically binds to a substance to be detected to a carrier by binding between biotin and a biotin-binding protein. In the present invention, "biotin and a biotin-binding protein" may be referred to as "avidin-biotin binding" in some cases.

Any protein that strongly binds to biotin can be preferably used as the biotin-binding protein, and examples thereof include avidin, streptavidin, neutravidin, AVR protein (Biochem. J., (2002), 363: 609-617), bradavidin (J. Biol. Chem., (2005), 280: 13250-13255), rhizavidin (Biochem. J., (2007), 405: 397-405), tamavidin (WO2002/072817), and variants thereof. The dissociation constant (KD) with biotin is preferably 10⁻⁶ M or less, more preferably 10⁻⁸ M or less, more preferably 10⁻¹⁰ M or less. Note that the biotin-binding protein that is added to a substance to be tested and the biotin-binding protein that is used for blocking the carrier will be described below.

Particularly preferred biotin-binding proteins are tamavidin and variants thereof, which can be highly expressed in E. coli. Tamavidin is a biotin-binding protein discovered in an edible mushroom, *Pleurotus cornucopiae* (W02002/072817, Takakura et al., (2009), FEBS J, 276: 1383-1397). An example of the variants of tamavidin is tamavidin exhibiting high binding capability and low non-specific binding characteristics (PCT/JP2009/64302).

The term "tamavidin" in the present invention refers to tamavidin 1, tamavidin 2, or a variant thereof. Specifically, tamavidin of the present invention may be typically a protein having the amino acid sequence of SEQ ID NO: 5 or SEQ ID NO: 7, or a protein encoded by a nucleic acid having the nucleotide sequence of SEQ ID NO: 4 or SEQ ID NO: 6. Alternatively, tamavidin of the present invention may be a protein that is a variant of a protein having the amino acid sequence of SEQ ID NO: 5 or SEQ ID NO: 7 or a protein encoded by a nucleic acid having the nucleotide sequence of SEQ ID NO: 4 or SEQ ID NO: 6 and having biotin binding capability similar to that of tamavidin 1 or 2 or high binding capability and low non-specific binding characteristics. Throughout the specification, tamavidin 1, tamavidin 2, and variants thereof may be collectively referred to as tamavidin.

The variant of tamavidin 1 or 2 may be a protein having an amino acid sequence comprising one or more deletion, substitution, insertion, and/or addition of one or more amino acids in the amino acid sequence of SEQ ID NO: 5 or 7 and having biotin binding capability similar to that of tamavidin 1 or 2. The substitution may be conservative substitution. The conservative substitution refers to replacement of a specific amino acid residue with any residue having similar physicochemical features. Nonlimiting examples of the conservative substitution include substitutions between amino acid residues containing aliphatic groups, such as mutual substitution between Ile, Val, Leu, and Ala; and substitutions between polar residues, such as mutual substitution between Lys and Arg, between Glu and Asp, and between Gln and Asn.

The variant by deletion, substitution, insertion, and/or addition of an amino acid or amino acids can be produced by a known technique such as site-specific mutagenesis (e.g., see Nucleic Acid Research, Vol. 10, No. 20, pp. 6487-6500, 1982, which is incorporated herein in its entirety) to a DNA encoding a wild-type protein. Throughout the specification, the term "one or more amino acids" herein refers to an amino acid or amino acids that can be deleted, substituted, inserted, and/or added by preferably site-specific mutagenesis. In addition, the term "one or more amino acids" herein may refer to one or more amino acids. The "one or several amino acids" refers to, but not limited to, 50 or less, preferably 40 or less, 30 or less, 20 or less, 10 or less, 8 or less, 5 or less, or 3 or less amino acids. The variant of tamavidin 1 or 2 may also be a protein having an amino acid sequence sharing an identity of 60% or more, preferably 65% or more, 70% or more, 75% or more, 80% or more, 85% or more, 90% or more, 95% or more, 96% or more, 97% or more, 98% or more, or 99% or more, more preferably 99.3% or more with that of SEQ ID NO: 5 or SEQ ID NO: 7 and having biotin binding capability similar to that of tamavidin 1 or 2 or high binding capability and low non-specific binding characteristics.

The percent identity between two amino acid sequences may be determined by visual inspection and mathematical calculation. Alternatively, the percent identity between two protein sequences may be determined through comparison of sequence information using a GAP computer program available from the University of Wisconsin Genetics Computer Group (UWGCG) based on the algorithm by Needleman, S. B. and Wunsch, C. D. (J. Mol. Biol., 48: 443-453, 1970). Preferred default parameters of the GAP program include: (1) scoring matrix: blosum62 described in Henikoff, S. and Henikoff, J. G., (Proc. Natl. Acad. Sci. USA, 89: 10915-10919, 1992); (2) 12 gap weights; (3) 4 gap length weights; and (4) no penalty for terminal gaps.

Any other program used by persons skilled in the art may also be used for comparison of the sequences. The percent identity can be determined by, for example, comparison with the sequence information using a BLAST program described in Altschul et. al., (Nucl. Acids. Res., 25, pp. 3389-3402, 1997). This program is available from the websites of National Center for Biotechnology Information (NCBI) or DNA Data Bank of Japan (DDBJ) on the Internet. The conditions (parameters) for identity search by the BLAST program is described in detail on these sites. Although these parameters can be partly modified if necessary, search is generally carried out using the default values. Alternatively, the percent identity between two amino acid sequences may be determined using a program such as genetic information processing software GENETYX Ver. 7 (available from GENETYX CORPORATION) or FASTA algorithm, wherein search may be carried out using the default values.

The percent identity between two nucleotide sequences can be determined by visual inspection and mathematical calculation. Preferably, such comparison is carried out through comparison of sequence information using a computer program. A particularly preferred computer program is a version 10.0 program "GAP", Wisconsin package of Genetics Computer Group (GCG, Madison, Wisconsin) (Devereux, et al., 1984, Nucl. Acids Res., 12: 387). The use of the "GAP" program enables comparison between two amino acid sequences and comparison between a nucleotide sequence and an amino acid sequence, in addition to comparison of two nucleotide sequences.

The "biotin-binding protein" constituting a fusion protein to be immobilized to a carrier is used for preparing a fusion protein-bound carrier by binding the fusion protein to the carrier through binding between biotin and the biotin-binding protein. Accordingly, it is preferred that the biotin binding activity of a variant of tamavidin 1 or 2 be not significantly decreased compared to that in the case of forming the fusion proteins using these wild-types.

Accordingly, nonlimitingly, in the variant of tamavidin 1, preferably, N14, S18, Y34, S36, S78, W82, W98, W110, and D118 in the amino acid sequence of SEQ ID NO: 5 are not modified. Note that the notation, for example, Y34 indicates the 34th tyrosine residue of the amino acid sequence of SEQ ID NO: 5. Alternatively, in the modification of these amino acid, the amino acid is preferably replaced with one having a similar property or structure. For example, asparagine (N14) is replaced with glutamine (Q) or aspartic acid (D), preferably aspartic acid; serine (S 18, S36, or S78) is replaced with threonine (T) or tyrosine (Y), preferably threonine; tyrosine (Y34) is replaced with serine (S), threonine (T), or phenylalanine (F), preferably phenylalanine; tryptophan (W82, W98, or W110) is replaced with phenylalanine (F); and aspartic acid (D118) is replaced with glutamic acid (E) or asparagine (N), preferably asparagine.

In the variant of tamavidin 2, preferably, four tryptophan residues (W69, W80, W96, and W108) in the amino acid sequence of SEQ ID NO: 7 are not modified. Alternatively, when these amino acid residues are modified, the amino acid is preferably replaced with one having a similar property or structure, for example, phenylalanine(F). In addition, it is desirable that amino acid residues (N14, S18, Y34, S36, S76, T78, and D116) that probably interact directly with biotin are also not modified. Alternatively, in the modification of these amino acid residues, the amino acid is preferably replaced with one having a similar property or structure in order to maintain the binding with biotin. For example, asparagine (N14) is replaced with glutamine (Q) or aspartic acid (D), preferably aspartic acid; aspartic acid (D40) is replaced with asparagine (N); serine (S18, S36, or S76) is replaced with threonine (T) or tyrosine (Y), preferably threonine; tyrosine (Y34) is replaced with serine (S), threonine (T), or phenylalanine (F), preferably phenylalanine; threonine (T78) is replaced with serine (S) or tyrosine (Y), preferably serine; and aspartic acid (D116) is replaced with glutamic acid (E) or asparagine (N), preferably asparagine.

Preferred variants of tamavidin in the present invention include the following variants (PCT/JP2009/64302).

The tamavidin variant is a modified biotin-binding protein that has the amino acid sequence of SEQ ID NO: 7 or an amino acid sequence comprising one to several amino acid mutations in this sequence or having an identity of at least 80% with this sequence and that shows biotin binding activity, wherein one or more residues selected from the group consisting of:
1) the arginine residue at the 104th site of SEQ ID NO: 7;
2) the lysine residue at the 141st site of SEQ ID NO: 7;
3) the lysine residue at the 26th site of SEQ ID NO: 7; and
4) the lysine residue at the 73rd site of SEQ ID NO: 7;
are replaced with acidic or neutral amino acid residues.

More preferably, the modified biotin-binding protein is selected from the group consisting of:
a modified biotin-binding protein (R104E-K141E) in which the arginine residue at the 104th site is replaced with a glutamic acid residue, and the lysine residue at the 141st site is replaced with a glutamic acid residue, in SEQ ID NO: 7;
a modified biotin-binding protein (D40N-R104E) in which the aspartic acid residue at the 40th site is replaced with a asparagine residue, and the arginine residue at the 104th site is replaced with a glutamic acid residue, in SEQ ID NO: 7;
a modified biotin-binding protein (D40N-K141E) in which the aspartic acid residue at the 40th site is replaced with a asparagine residue, and the lysine residue at the 141st site is replaced with a glutamic acid residue, in SEQ ID NO: 7; and
a modified biotin-binding protein (D40N-R104E-K141E) in which the aspartic acid residue at the 40th site is replaced with a asparagine residue, the arginine residue at the 104th site is replaced with a glutamic acid residue, and the lysine residue at the 141st site is replaced with a glutamic acid residue, in SEQ ID NO: 7.

### Fusion protein between protein specifically binding to substance to be detected and biotin-binding protein

In the present invention, a fusion protein between a protein that specifically binds to a substance to be detected and a biotin-binding protein (hereinafter may be referred to as "biotin-binding protein fusion protein" or "fusion protein") is immobilized on a carrier.

The method for preparing the biotin-binding protein fusion protein is not particularly limited. For example, the fusion protein may be expressed by a known genetic engineering technique. For example, the fusion protein can be obtained by expressing a gene encoding a fusion protein between a biotin-binding protein and a desired protein using an expression system such as E. coli. Note that tamavidin and variants thereof are preferred to achieve a high expression in E. coli.

In the biotin-binding protein fusion protein, a biotin-binding protein and a desired protein may be directly bound to each other or may be bound via a linker, but are preferably bound via an amino acid linker. The length of the linker may be at least one amino acid, but is preferably at least five amino acids and more preferably at least six amino acids. In order to enhance the binding strength between biotin immobilized on a carrier and tamavidin, the length of the linker is preferably at least ten amino acids, more preferably at least 12 amino acids, at least 15 amino acids, at least 18 amino acids, and most preferably at least 25 amino acids. Probably, such linkers also increase the activity of the tamavidin fusion protein. The amino acids constituting the linker are not particularly limited, and the linker is preferably made of repetition of neutral amino acids such as glycine, serine, or alanine. Nonlimiting examples thereof include GGGGS, GGSGG, GASAG, GSGAA, GSGSA, GGGGSG, GGGSGGS, GGSGGGGS, AAAAGSGAA, GGGGSGGGGSGGGGS, and GGGGSGGGGSGGGGSGGGGSGGGGS (SEQ ID NOs: 8 to 18).

The biotin-binding protein may be bound to either the N-terminal or the C-terminal of a desired protein. In the expression of a desired protein, for example, if the periplasmic space is more suitable than the cytoplasm of E. coli, a leader sequence for targeting the periplasm may be used. Examples of the leader sequence include, but not limited to, PelB (Lei et al., (1987), J Bacteriol, 169: 4379-4383) and OmpA (Gentry-Weeks et al., (1992), J Bacteriol, 174: 7729-7742).

In the case of a biotin-binding protein fusion protein obtained from a soluble fraction, the soluble fraction, without purifying the crude protein extract, may be put into direct contact with a biotinylated carrier to bind the fusion protein to the biotinylated carrier. Through sufficiently washing the carrier, the purification and the immobilization to the carrier of the fusion protein can be simultaneously achieved. Alternatively, the fusion protein may be bound to the biotinylated carrier after purification using a column to which a biotin analog such as iminobiotin Hofmann et al., (1980), Proc Natl Acad Sci USA, 77: 4666-4668) is bound.

Alternatively, a tag for purification may be further added to the N-terminal or the C-terminal of the biotin-binding protein fusion protein. Examples of the tag include, but not limited to, a c-myc epitope tag (Munro and Pelham, (1986), Cell, 46: 291-300), a histidine tag (Hochuli et al., (1988), Bio/Technol, 6: 1321-1325; Smith et al., (1988), J Biol Chem, 263: 7211-7215), a Halo tag (Los and Wood, (2007), Methods Mol Biol, 356: 195-208), a Flag tag (Einhauer and Jungbauer, (2001), J Biochem Biophys Methods, 49: 455-465), and combinations thereof.

In the case of a biotin-binding protein fusion protein obtained from an insoluble fraction, a known process employed, for example, the protein is once solubilized using a chaotropic salt such as urea or guanidine hydrochloride, and then refolding of the protein is enhanced with gradually removing the chaotropic salt by, for example, dialysis (Sano and Cantor, (1991), Bio/Technology, 9: 1378-1381; Sano et al., (1992), Proc Natl Acad Sci USA, 89: 1534-1538).

In the case of a desired protein expressed in an insoluble fraction in E. coli, for example, a maltose-binding protein (Bach et al., (2001), J Mol Biol, 312: 79-93), thioredoxin (Jurado et al., (2006), J Mol Biol, 357: 49-61), glutathione S-transferase (Tudyka and Skerra, (1997), Protein Sci, 6: 2180-2187), or a chaperon such as one described in Ideno et al., (2004), Appl Microbiol Biotechnol, 64: 99-105 may be coexpressed, or a three-component fusion protein composed of the fusion protein and a chaperon may be produced. The maltose-binding protein, the thioredoxin, and the glutathione S-transferase can also be used as tags for purification.

The fusion protein may be expressed by any other known expression system, such as insect cells, plant cells, mammalian cells, yeast cells, Bacillus subtilis cells, or a cell-free expression system. In particular, when the protein to be fused is expressed in plant cells (for example, plant lectin), it is preferred that the fusion protein is also expressed in a plant cell expression system. A suitable expression system will be apparent to those skilled in the art in consideration of properties of the protein to be fused.

### Binding of fusion protein to carrier

In the present invention, a protein can be bound to a carrier through avidin-biotin binding by providing a biotin-bound carrier and a biotin-binding protein fusion protein and putting them into contact with each other.

A crude cell homogenate extract containing a biotin-binding protein fusion protein is prepared in a total protein content of, but is not limited to, 0.1 mg/mL to 5 mg/mL, preferably 0.2 mg/mL to 2 mg/mL. This extract is put into contact with a carrier to which biotin is bound at 10°C to 40°C, preferably 20°C to 30°C, for 5 min to 2 hr, preferably 30 min to 1 hr. Alternatively, a purified biotin-binding protein fusion protein in a concentration of 0.1 µg/mL to 5 µg/mL may be put into contact with a carrier to which biotin is bound.

### Addition of biological sample to carrier (step 3 of Embodiment 1)

The method of Embodiment 1 of the present invention includes, after the providing of a fusion protein-bound carrier, the following step 3):
mixing
   (a) a biological sample, and
   (b-i) a cell homogenate extract prepared from cells of the same species as the host cells used for expressing the fusion protein in step 1), and a biotin-binding protein, or
   (b-ii) a cell homogenate extract prepared from cells of the same species as the host cells used for expressing the fusion protein in step 1), and genetically engineered to express a biotin-binding protein,
and adding the mixture to the fusion protein-bound carrier produced in step 2).

In a method of detection using a carrier, generally known approaches for reducing non-specific binding which causes background signals are, for example, addition of an extract of a bacterium component to a reagent for detection (JP No. S59-99257 A (1984)); addition of a culture component of host cells to a sample, where a vector of the same species as that used in production of a recombinant protein capable of specifically binding to a substance to be detected and containing no gene encoding the protein (JP No. H8-43392 A (1996)); heat treatment of an aqueous extract from cells of the same species as that producing a recombinant protein capable of specifically binding to a substance to be detected and not containing this protein, and addition of its water-soluble fraction to a sample (JP No. 2004-301646 A).

The present inventors had attempted to apply the above-mentioned methods to a system of fusion protein, but sufficient effect has was not obtained. However, the present inventors have diligently studied and, as a result, have arrived at a method of obtaining a noticeable effect. Specifically, the present inventors have discovered that it is preferable to put a substance to be detected into contact with a carrier in the presence of both a cell homogenate extract and a biotin-binding protein.

In one embodiment, a cell homogenate extract prepared from cells of the same species as the host cells used for expressing the fusion protein, biotin-binding protein, and a biological sample are mixed, and the mixture may be added to a carrier. When a biotin-binding protein and a cell homogenate extract are added to a sample to be tested, either one may be added first, or the both may be simultaneously added. Alternatively, after mixing the biotin-binding protein and the cell homogenate extract, the sample to be tested may be added thereto.

In another embodiment, a cell homogenate extract prepared from cells of the same species as the host cells used for expressing the fusion protein and genetically engineered to express a biotin-binding protein and a sample to be tested are mixed, and the mixture may be added to a carrier. Specifically, a recombinant protein is expressed in cells by integrating the biotin-binding protein into an expression vector, and the homogenate extract from the cells may be used.

In the case of a serum sample to be tested, the serum is usually used after dilution in a cell homogenate extract into 10 to 10000-fold, preferably 100 to 1000-fold, more preferably 100 to 500-fold.

Cells from which cell homogenate extracts are derived, such as E. coli cells, yeast cells, mammalian cells, insect cells, and plant cells, can be used without limitation. Preferred cells are the same species as that of host cells used to express a fusion protein.
For example, in the case of preparation of a fusion protein using E. coli, it is preferred that the cell homogenate extract be also prepared using E. coli. In the case of expression of a fusion protein in a cell-free system, the cell homogenate extract used can be used as it is or as a suspension in a desired buffer solution.

More preferably, the cells are derived from different species from the organism from which the biological sample is derived. For example, for a biological sample derived from human being, a cell homogenate extract is preferably prepared from cells derived from an organism other than human beings. However, for example, for a fusion protein expressed in mammalian cells, a cell line obtained from specific cancer cells is generally used. In such a case, the content of the human cell line and the content of (for example) human cells from which the biological sample are derived are substantially highly different from each other, even if the cell homogenate extract from a cell line derived from human being is used; hence, the effect of the present invention can be obtained.

The cell used for preparation of the cell homogenate extract may contain any vector, preferably an empty vector. The empty vector is of the same species as that of the vector used in expression of a protein that specifically binds to substance to be detected or a fusion protein thereof and does not contain genes that encode these proteins. The empty vector may further contain any nucleic acid. Alternatively, the empty vector may be any known vector that is different from the vector used in expression of a protein that specifically binds to substance to be detected or a fusion protein thereof.

Any cell homogenate extract derived from cells can be used without limitation. For example, a protein component, a carbohydrate component, or a lipid component of cells, or a mixture thereof may be used. Preferably, a soluble extract of cells can be used.

The cell homogenate extract can be prepared by a variety of processes without limitation. Usually, the cell homogenate extract can be prepared as a soluble components by homogenizing or solubilizing cells cultured in a proper culture medium through a physical means such as, ultrasonic application, a chemical means using a surfactant, or an enzymatic treatment, and performing centrifugation or filtration. In order to prolong the storage life, preferably the clear liquid prepared by centrifugation or filtration is mixed with, for example, a protease inhibitor, or is heated, for example, in an autoclave to suppress or deactivate various enzymes derived from cells. The concentration of the cell homogenate extract can be varied depending on the extent of the occurring non-specific reaction and can be set in a range sufficient to absorb the non-specific reaction.

As a nonlimiting specific example of the method for preparing a cell homogenate extract, in the case of E. coli cells, E. coli cells (which may contain a vector optionally containing a gene encoding a biotin-binding protein) are inoculated into an LB culture medium containing an antibiotic; shake culture is performed at 25°C to 37°C until the absorbance at OD 600 reaches 0.25 to 1, preferably 0.4 to 0.6; 0.1 mM to 5 mM, preferably 0.5 mM to 1 mM IPTG is added thereto; and then shake culture is performed at 25°C to 37°C for 2 to 24 hr, preferably 4 to 16 hr. The bacterial cells are recovered by centrifugation from the culture solution, are suspended in a desired buffer solution, and are homogenized. After centrifugation of the homogenized solution, the supernatant is collected as a crude E. coli extract.

The biological sample and cell homogenate extract can be added to a carrier by any procedure. The biological sample, however, must come into contact with the cell homogenate extract before the contact with the carrier. In other words, as long as the biological sample is put into sufficient contact with the cell homogenate extract, the component derived from the cell homogenate extract is not necessarily added to the carrier finally together with the biological sample. For example, a carrier to which the cell homogenate extract component is bound is prepared, and a treated biological sample may be added thereto. Specific examples include an embodiment in which a biological sample is passed through a cell homogenate extract component column.

When a biological sample is mixed with a crude cell homogenate extract, the sample is reacted with, but not limited to, a crude cell homogenate extract that has been prepared with a desired buffer (which may contain, for example, BSA, casein, or a commercially available blocking agent) into a total protein concentration of 0.05 to 5 mg/mL, preferably 0.5 to 5 mg/mL, at 10°C to 30°C, preferably, 20°C to 30°C for 30 min to 4 hr, preferably 1 to 2 hr. For a biological sample of serum, the serum is preferably diluted with, but not limited to, the crude cell homogenate extract to 100 to 1000-fold.

### Addition of biotin-binding protein to sample to be tested

The present invention is **characterized in that** a mixture of a biological sample, a cell homogenate extract prepared from cells of the same species as the host cells used for expressing a fusion protein, and a biotin-binding protein is added to a carrier (step b-i), or a mixture of a sample to be tested and a cell homogenate extract prepared from cells of the same species as the host cells used for expressing a fusion protein and genetically engineered to express a biotin-binding protein is added to a carrier (step b-ii).

In the method of the present invention, the background signal level can be finally suppressed by adding a biotin-binding protein to a sample to be tested.

Such a biotin-binding protein may be the same as or different from the biotin-binding protein constituting a fusion protein. Either the wild type or a variant may be used. The biotin-binding activity of the variant may be equivalent, higher, or lower compared to that of the wild type.

In an embodiment of the addition, powder of a biotin-binding protein (which may be a naturally occurring protein or may be expressed by genetic engineering) may be added to the sample directly or after its dissolution in a proper liquid. For example, in another embodiment, a mixture of a sample and a cell homogenate extract may be treated with a carrier to which the biotin-binding protein is immobilized (for example, the mixture is passed through a column) (step b-i), instead of direct addition of the biotin-binding protein to the sample.

In the case of addition of the biotin-binding protein to a crude cell homogenate extract, the final concentration of the added biotin-binding protein is, but not limited to, 1 to 500 µg/mL, preferably 10 to 100 µg/mL. The concentration of the biotin-binding protein that is genetically engineered to be expressed in the cells may also substantially be the same as above, but any other concentration is acceptable.

Alternatively, a gene encoding a biotin-binding protein is introduced into host cells and is expressed, and a cell extract containing a biotin-binding protein obtained by homogenizing the host cells may be used (step b-ii). In this case, the biotin-binding protein may be expressed in a desired host by a method well known to persons skilled in the art. In the case where a fusion protein between a protein that specifically binds to a substance to be detected and a biotin-binding protein is expressed by genetic engineering, the cell homogenate extract is preferably derived from the same species as that of the host.

In the case of the host being E. coli, a gene encoding the biotin-binding protein is incorporated into an expression vector and is introduced into E. coli, and E. coli is cultivated while expression of protein is induced. Conditions for induction, such as an expression vector and host E. coli strains, culture medium component, IPTG concentration, and cultivation temperature can be appropriately determined.

### Method of detection of substance to be detected (step 4 of Embodiment 1)

The method of detection according to the present invention detects a substance bound to the protein that specifically binds to a substance to be detected in a fusion protein.

Persons skilled in the art can appropriately select the method for detecting substance to be detected based on the properties of a desired protein. Preferred examples of such a method include immunoassays such as enzyme-linked immunosorbent assay (ELISA, including sandwich ELISA)) and radioimmunoassay (RIA); and other assays such as nucleic acid hybridization assay and surface plasmon resonance. After a sample to be tested is reacted with a substance that specifically binds and interacts with a substance to be detected and is immobilized by avidin-biotin binding, and the substance is detected.

In the immunoassay, for example, of a substance to be detected of an antibody, an antigen is immobilized and is reacted with the antibody present in a sample to be tested, and the reaction product is detected by any method well known to persons skilled in the art. For example, in the case where the sample to be tested is one derived from human being, the human antibody bound to the antigen is detected using an anti-human antibody. In this procedure, the anti-human antibody is labeled with a phosphor, enzyme, or radioisotope, and the fluorescence intensity, enzyme activity, or radiation dose is finally measured to indirectly measure or determine the amount of the antibody. When the substance to be determined is an antigen, an antibody against one site (epitope) of the antigen is immobilized and is reacted with the antigen present in a sample to be tested, and an antibody against another epitope of the antigen is further reacted with the antigen. In this procedure, this secondary antibody against the other epitope is labeled as described above to indirectly measure the amount of the antigen. Alternatively, in the nucleic acid hybridization assay, a nucleic acid composed of several tens of nucleotides to several hundreds or several thousands nucleotides and having a sequence region complementary to a nucleic acid to be determined is immobilized by avidin-biotin binding. This is reacted with a sample to be tested containing a nucleic acid labeled with a phosphor or radioisotope, and the amount of the phosphor or isotope is measured.

Any labeling well known to persons skilled in the art may be employed. Alternatively, commercially available phosphor- or enzyme-labeled anti-human antibodies may be used. Examples of the phosphor labeling include labeling using, for example, fluorescein or rhodamine, and labeling using a fluorescent protein such as a green fluorescent protein (GFP). Enzymes used for enzyme labeling are, but not limited to, peroxidase, alkaline phosphatase, luciferase, and glucose oxidase. Substrates for measurement using these enzymes are commercially available. For example, TBA and substrates for chemiluminescence can be used for peroxidase. Examples of the radioisotope include iodine (¹²⁵I and ¹²¹I), carbon (¹⁴C), sulfur (³⁵S), and tritium (³H), and phosphorus (³²P) for nucleic acids.

The amount of an antigen or an antibody present in the biological sample (specimen) can be readily calculated by comparison with the amount present in a standard preparation (e.g., a standard sample of a healthy subject or a typical patient in the case of clinical samples) using a linear regression computer algorithm. Such assay for detecting an antigen or an antibody, for example, ELISA is disclosed in Iacobelli et al., Breast Cancer Research and Treatment, 11: 19-30 (1988).

For example, in the case that the amount of the substance to be detected, e.g., antibody, in a biological sample is small (the case of a low antibody titer) or that the number of non-specific binds due to a biological sample, such as serum, per se is large, the effect of background signals due to non-specific binding would be noticeable. Accordingly, the substance to be detected can be more precisely determined by appropriately subtracting the background signals from the measured value. Those skilled in the art can appropriately determine the background signal to be subtracted depending on each experimental system.

For example, an example of such an embodiment is a "Human/Monkey anti-type I and type II Collagen IgG Antibody assay kit" (manufactured by Chondrex), which detects an antibody against collagen present in serum. In this kit, the background value (measured value of the secondary antibody only without addition of serum) is subtracted from the measured value of a sample.

In the case where the number of non-specific binds due to a biological sample per se is large, such as in case of serum, , in order to subtract the background due to the non-specific reaction, as described in Example 2, the following embodiment is effective. The measured value in the section where an SITH-1 antigen (protein that specifically binds to the substance to be detected) is not immobilized (however, in the section, blocking with BSA or the like has been operated, and the serum (biological sample) containing the anti-SITH-1 antibody (the substance to be detected in the biological sample) has been added, as in the section where the SITH-1 antigen is immobilized) is subtracted from the measured value at the carrier (plate) on which a fusion protein between the SITH-1 antigen and tamavidin is immobilized. Alternatively, as described in Example 2 or 3, the amount may be calculated by subtracting the measured value in a carrier where only tamavidin is immobilized (bound) is subtracted from the measured value in a carrier (plate) where a fusion protein between an SITH-1 antigen and tamavidin is immobilized.

In another example of the embodiment for subtracting non-specific reaction specific to sample per se, as the above-mentioned "Human/Monkey anti-type I and type II Collagen IgG Antibody assay kit", the measured value at a well on which collagen is not immobilized may be subtracted from the measured value of a serum collagen antibody at a well of a microtiter plate on which collagen is immobilized. Furthermore, a kit of a similar embodiment is "Virus antibody EIA "Seiken"Herpes IgM" (manufactured by Denka Seiken) that is used for detecting an anti-simplex herpes virus IgM antibody in serum or plasma.

Preferably, the amount can be more precisely determined by subtracting the measured value in the section where an arbitrary protein of which antibody is not possessed by an organism (e.g., human being in the case of SITH-1) from which the sample is derived is immobilized (a non-limiting example of the immobilized protein is GFP, when the organism is a mammalian). The method of immobilization is not particularly limited, and, preferably, a fusion protein with a biotin-binding protein is produced and is immobilized on a biotinylated carrier by avidin-biotin binding.

Persons skilled in the art can appropriately design and select the method of calculation as in above based on the properties of a biological sample and the characteristics of an antibody used.

The method of detection according to the present invention can specifically detect an antibody having a low antibody titer in, preferably, serum.

The effect of Embodiment 1 of the present invention can be apparent by, for example, comparing the results of TM2/pTrc99A/BL21 cell homogenate extract (closed triangle) in Fig. 3-1 with the results of PBS (open square) or pTrc99A/BL21 cell homogenate extract (closed circle) in Example 2. Furthermore, the effect can be also apparent by comparing the S/N ratio of the crude extract of BL2 introduced with TM2/pTrc99A with the S/N ratio of PBS or the crude cell homogenate extract of BL2 introduced with pTrc99A shown in Table 3 in Example 3.

### II. Method of detection of the present invention (Embodiment 2)

The method of detection (Embodiment 2) according to the present invention relates to a method for detecting a substance in a biological sample and comprises:
1) providing a carrier on which biotin is bound and providing a fusion protein between a protein that specifically binds to a substance to be detected and a biotin-binding protein;
2) binding the fusion protein to the carrier provided in step 1) through binding between biotin and the biotin-binding protein to produce a fusion protein-bound carrier;
3) putting a biotin-binding protein into contact with the fusion protein-bound carrier produced in step 2) to block the carrier;
4) after the blocking step in step 3), adding a biological sample to the fusion protein-bound carrier; and
5) detecting a substance to be detected that has bound to the protein that specifically binds to the substance in the fusion protein.

In Embodiment 2 of the present invention, the method preparing the fusion protein-bound carrier in steps 1 and 2 is the same as that in Embodiment 1. In addition, the last step of detecting a substance to be detected that has been bound to a protein specifically binding to a substance to be detected in the fusion protein (step 4 of Embodiment 1 and step 5 of Embodiment 2) is the same as that in Embodiment 1.

The method of Embodiment 2 is characterized by a step of blocking a carrier (step 3 of Embodiment 2) before addition of a biological sample to the carrier.

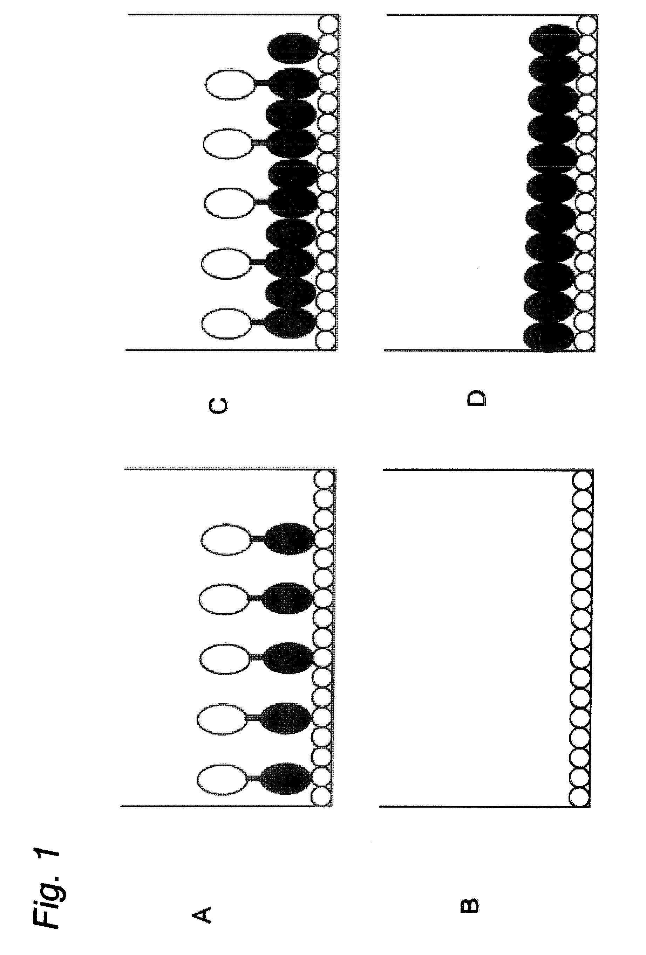

In the present invention, background signals can be further suppressed by putting a biotin-binding protein into contact with a fusion protein-bound carrier before the addition of a biological sample to the carrier. It is believed that the biotin-binding protein binds to the biotin moiety of the fusion protein in a free state without binding on the carrier surface (Fig. 1), though not necessarily restricted by a theory.

The biotin-binding protein that will be put into contact with the binding carrier may be the same as or different from the biotin-binding protein constituting the fusion protein, and may be either a wild type or a variant. The biotin-binding activity of the variant may be equivalent, higher or lower compared to that of the wild type, but is preferably equivalent or lower. The variant may be, for example, but not limited to, a protein that has a biotin-binding activity lower than that of the wild-type protein and has a dissociation constant (KD) with biotin of 10⁻⁵ (M) or less. Such a biotin-binding protein can be produced by chemical modification (US-A-5051356), but a method of modifying an amino acid sequence is most preferred (Qureshi et al., (2002), J. Biol. Chem., 276: 46422-46428).

In the present invention, the biotin-binding protein may be put into contact with the binding carrier by any method without particular limitation. That is, the biotin-binding protein may be added to a carrier directly or in a form of an appropriate solution. Furthermore, a blocking agent well-known to persons skilled in the art, such as BSA or casein, may be simultaneously used.

In a nonlimiting example, a biotin-binding protein is added to a desired buffer (e.g., TBS or PBS containing 0.02% to 1%, preferably 0.1% to 0.5%, of Tween-20 or Triton) in a final concentration of 1 to 500 µg/mL, preferably 10 to 100 µg/mL. BSA or casein may be further added thereto in a final concentration of 1 to 50 µg/mL, preferably 5 to 10 µg/mL. This blocking solution is put into contact with a carrier to which a biotin-binding protein fusion protein is bound by avidin-biotin binding at 10°C to 40°C, preferably 20°C to 30°C, for 10 min to 2 hr, preferably 30 min to 1 hr.

The effect of Embodiment 2 of the present invention can be apparent by, for example, in Example 2, comparing the value at antibody being 0 with the value in pTrc99A/BL21 cell homogenate extract (closed circle) shown in Figs. 3-1 and 3-2. At antibody being 0, no antibody is present; hence, the fluorescence would be 0 if non-specific binding does not occur. Here, the comparison of the values at antibody being 0 shown by closed circles in Figs. 3-1 and 3-2 confirms that the fluorescence value is reduced to almost a half in the case of blocking of a carrier with TM2 and is apparent that the non-specific binding is considerably reduced. In Fig. 3-2 showing the result of blocking of a carrier with TM2, the graph overall shifts below compared to that in Fig. 3-1. Furthermore, the effect can be apparent by comparing the value in "blocking by BSA" and the value in "blocking by BSA and TM2" for the results of pTrc99A/BL21 cell homogenate extract in Table 2 of Example 2.

### III. Method of detection of the present invention (Embodiment 3)

The method of detection (Embodiment 3) according to the present invention relates to a method for detecting a substance in a biological sample and comprises:
1) providing a carrier on which biotin is bound and providing a fusion protein between a protein that specifically binds to a substance to be detected and a biotin-binding protein;
2) binding the fusion protein to the carrier provided in step 1) through binding between biotin and the biotin-binding protein to produce a fusion protein-bound carrier;
3) putting a biotin-binding protein into contact with the fusion protein-bound carrier produced in step 2) to block the carrier;
4) after the blocking step in step 3), mixing
   (a) a biological sample, and
   (b-i) a cell homogenate extract prepared from cells of the same species as the host cells used for expressing the fusion protein in step 1), and a biotin-binding protein, or
   (b-ii) a cell homogenate extract prepared from cells of the same species as the host cells used for expressing the fusion protein in step 1), and genetically engineered to express a biotin-binding protein,
   and adding the mixture to the fusion protein-bound carrier; and
5) detecting a substance to be detected that has bound to the protein that specifically binds to the substance in the fusion protein.

### Embodiment 3 is a combination of Embodiment 1 and Embodiment 2.

Specifically, the addition of a cell homogenate extract and a biotin-binding protein in Embodiment 1 and the blocking of a carrier with a biotin-binding protein in Embodiment 2 are performed.

The effect of Embodiment 3 of the present invention can be apparent by, for example, comparing the results of TM2/pTrc99A/BL21 cell homogenate extract (closed triangle) in Fig. 3-2 with other results in Figs. 3-1 and 3-2, for example, the results of PBS (open square) in Example 2. The effect can also be apparent by comparing the S/N ratio of the crude extract of BL21 introduced with TM2/pTrc99A with other results, for example, the S/N ratio of PBS, E. coli (BL21), or the crude cell homogenate extract of BL21 introduced with pTrc99A shown in Table 5 in Example 4.

### IV Fusion protein-bound carrier

The present invention also provides a carrier for detecting a substance in a biological sample. The carrier of the present invention is characterized by a fusion protein between a protein that specifically binds to a substance to be detected and a biotin-binding protein is bound to a carrier by binding between biotin and the biotin-binding protein, wherein the fusion protein is bound to the carrier by a method comprising:
1) providing a carrier on which biotin is bound and providing a fusion protein between a protein that specifically binds to a substance to be detected and a biotin-binding protein;
2) binding the fusion protein to the carrier provided in step 1) through binding between biotin and the biotin-binding protein to produce a fusion protein-bound carrier; and
3) putting a biotin-binding protein into contact with the fusion protein-bound carrier produced in step 2) to block the carrier. The carrier of the present invention is blocked with a biotin-binding protein before the addition of a biological sample.

Accordingly, in the carrier of the present invention, preferably, a fusion protein between a protein that specifically binds to a substance to be detected and a biotin-binding protein is bound to a carrier by avidin-biotin binding, and a biotin-binding protein is bound to unreacted biotin on the carrier by avidin-biotin binding.

### VII. Kit

The present invention also provides a kit for detecting a substance in a biological sample. The kit of the present invention comprises:
A) a carrier on which a fusion protein between a protein that specifically binds to a substance to be detected and a biotin-binding protein is bound by binding between biotin and the biotin-binding protein; and
   an agent for diluting a biological sample, comprising
B-i) a cell homogenate extract prepared from cells of the same species as the host cells used for expressing the fusion protein in step A), and a biotin-binding protein, or
B-ii) a cell homogenate extract prepared from cells of the same species as the host cells used for expressing the fusion protein in step A), and genetically engineered to express a biotin-binding protein; and/or
C) a blocking agent containing a biotin-binding protein.

The agent for diluting a biological sample may be a cell homogenate extract (and biotin-binding protein) itself or may be an agent for diluting a cell homogenate extract together with a biological sample and may contain an appropriate buffer and a solvent such as a commercially available cell diluent or serum diluent.

### EXAMPLES

The present invention will now be described specifically by way of the following examples, which are not intended to limit the technical scope of the invention. Based on the descriptions of the specification, modifications and changes will be apparent to those skilled in the art, and such modifications and changes fall within the technical scope of the invention.

In Examples 1 and 2, a fusion protein between a human herpes virus 6 (HHV-6)-derived SITH-1 protein and tamavidin 2 was expressed in E. coli, and an E. coli crude extract obtained by sonication was directly reacted with a biotinylated microplate to thereby bind the fusion protein to the microplate through tamavidin-biotin binding. The resulting SITH-1 plate was reacted with human serum diluted with an E. coli crude extract (supplemented with rabbit anti-SITH-1 antibody; in this test, commercially available human sera supplemented with serial dilutions of rabbit anti-SITH-1 antibody (antiserum) were used as pseudo-clinical specimens) to measure the titer of anti-SITH-1 antibody contained in the human serum.

### Example 1: SITH-1 gene and construction of vector for expression of fusion protein of tamavidin 2

In this example, a gene encoding a fusion protein having tamavidin 2 (TM2) located at the C-terminal side of the SITH-1 via a linker was designed.

### 1-1. Design of primer

In order to construct a SITH1-TM2 fusion gene, first, primers for fusion of SITH-1 and TM2 genes via a linker (5xlinker: GGGGSGGGGSGGGGSGGGGSGGGGS) (SEQ ID NO: 18) were designed.

That is, a primer consisting of a DNA sequence encoding a C-terminal region of SITH-1 at the 5'-side, a linker at the center, and an N-terminal region of TM2 at the 3'-side (SITH1C-5xlink-TM2N-F)(SEQ ID NO: 19) and a primer consisting of a DNA sequence encoding the N-terminal region of TM2 at the 5'-side, the linker, and the C-terminal region of SITH-1 at the 3'-side in the reverse direction (SITH1C-5xlink-TM2N-R) (SEQ ID NO: 20) were designed.

Then, a primer consisting of a 5' region including an N-terminal region of SITH-1 and an EcoRI restriction enzyme cleavage site (CCATGG) located upstream thereof (SITH1 5' EcoRI-F (SEQ ID NO: 21)) and a primer consisting of a sequence encoding a 3' region of the TM2 gene and a BamHI restriction enzyme cleavage site (GGATCC) located downstream thereof (TM2CtermBam) (SEQ ID NO: 22) were designed. The primers for construction of a fusion gene between SITH-1 and TM2 are summarized in Table 1.

**[Table 1]**

| Primers for construction of a fusion gene between SITH-1 and TM2 | | |
|---|---|---|
| Name | Sequence | Length |
| SITH1 5' EcoRI-F | AAAGAATTCGGATATGAAGAAAAAGTGTC | 29 mer |
| SITH1C-5xlink-TM2N-F | CCGAAAACTGAAATGAATGTGggtggcggtggcagc ggtggcggtggcagcggtggcggtggcagcggtggcggtggcagcgg tggcggtggcagcTCAGACGTTCAATCTTCACTC | 117 mer |
| SITH1C-5xlink-TM2N-R | GAGTGAAGATTGAACGTCTGAgctgccaccgccacc gctgccaccgccaccgctgccaccgccaccgctgccaccgccaccgct gccaccgccaccCACATTCATTTCAGTTTTCGG | 117 mer |
| TM2CtermBam | TTTGGATCCTTACTTCAACCTCGGTGCG | 28 mer |

The restriction enzyme recognition sites are underlined. The linker sequences are indicated in small letters.

### 1-2. PCR

In order to construct a SITH1-TM2 fusion gene, PCR was performed in two steps.

In the first step of PCR, a plasmid obtained by integrating the SITH-1 gene (ORF) (SEQ ID NO: 2) into FLAG Expression Vector (SIGMA) was used as a template to amplify a SITH-1 region with the primers SITH1 5' EcoRI-F and SITH1C-5xlink-TM2N-R, and a plasmid obtained by integrating the TM2 gene into vector pTrc99A (WO2002/072817) was used as a template to amplify a TM2 region with the primers SITH1C-5xlink-TM2N-F and TM2CtermBam.

PCR was accomplished using a GeneAmp PCR System 9600 (PERKIN ELMER) in 20 µL reaction solution containing template DNA (500 ng), 10 x ExTaq buffer (2 µL, TaKaRa), 2.5 mM dNTP (1.6 µL), primers (20 pmoles each), and 5 U/µL ExTaq (0.1 µL) under reaction conditions: 96°C for 3 min, (95°C for 1 min, 60°C for 1 min, 72°C for 2 min) x 20 cycles, and 72°C for 6 min. As a result, a PCR product of 579 bp was obtained for the SITH-1 region, while a PCR product of 528 bp was obtained for the TM2 region. These PCR products were fractionated in a TAE buffer using agarose gel electrophoresis. The gel was cut out for each PCR product, and the products were collected using a QIAEX II gel extraction kit (QIAGEN). The extraction was performed according to the manual attached to the kit.

The two PCR products were used as templates to perform the second step of PCR using the primers SITH1 5' EcoRI-F and TM2CtermBam. The reaction was accomplished using a GeneAmp PCR System 9600 (PERKIN ELMER) in 20 µL reaction solution containing template DNA (500 ng), 10 x Pyrobest buffer (2 µL, TaKaRa), 2.5 mM dNTP (1.6 µL), primers (20 pmoles each), and 5 U/µL Pyrobest DNA polymerase (0.1 µL) under reaction conditions: 96°C for 3 min, (95°C for 1 min, 60°C for 1 min, 72°C for 2 min) x 20 cycles, and 72°C for 6 min. As a result, a PCR product of 990 bp was obtained.

### 1-3. Cloning

The SITH1-TM2 fusion gene obtained by PCR was cloned into vector pCR4Blunt TOPO (Invitrogen).

Specifically, first, ligation reaction was performed according to the manual attached to the vector kit. The DNA was introduced into E. coli TB1 by electroporation, and the plasmid DNA was extracted in a routine manner (Sambrook et al., 1989, Molecular Cloning, A laboratory manual, 2nd edition). Each plasmid for which the presence of an insert was confirmed was analyzed with M13 primers (TaKaRa) and an ABI PRISM fluorescent sequencer (Model 310 Genetic Analyzer, Perkin Elmer) to determine its nucleotide sequence, which was then confirmed to have no mutation in comparison with the sequence of the designed gene. The plasmid carrying the fusion gene was double-digested with EcoRI and BamHI, and agarose gel electrophoresis and purification were performed in the same manner as described above to collect a DNA fragment containing the fusion gene. This fragment was ligated into E. coli expression vector pTrc99A (Pharmacia), which had been digested with EcoRI and BamHI, using a Ligation Kit (TaKaRa). In the manner described above, a vector for SITH1-TM2 fusion protein expression, SITH1-TM2/pTrc99A, was completed. The ligation product was transformed into E. coli TB 1, and the plasmid DNA was extracted and was further transformed into E. coli BL21. The resulting E. coli colonies were each used as a template to amplify an insert gene region by PCR with SITH1 5' EcoRI-F and TM2CtermBam to thereby confirm the presence or absence of the insert gene.

In the manner described above, a vector SITH1-TM2/pTrc99A for expression of the fusion protein between SITH1 and TM2 was completed. The nucleotide sequence encoding SITH1-TM2 in the expression vector SITH1-TM2/pTrc99A is shown in SEQ ID NO: 23, and the amino acid sequence encoded thereby is shown in SEQ ID NO: 24.

In the 5' side of SITH1-TM2, two amino acid residues are added downstream of the translation initiation methionine (nucleotides 4-9 in SEQ ID NO: 23, amino acid residues 2-3 in SEQ ID NO: 24) because the EcoRI site was used for integration into pTrc99A. These amino acid residues are followed by the SITH-1 sequence (nucleotides 10-483 in SEQ ID NO: 23, amino acid residues 4-161 in SEQ ID NO: 24), 5xlinker (nucleotides 484-558 in SEQ ID NO: 23, amino acid residues 162-186 in SEQ ID NO: 24), and the TM2 sequence (excluding Met) (nucleotides 559-981 in SEQ ID NO: 23, amino acid residues 187-326 in SEQ ID NO: 24).

### 1-4. E. coli expression

E. coli BL21 carrying SITH1-TM2/pTrc99A was inoculated into LB medium (50 mL) containing an antibiotic, ampicillin, (final concentration: 100 µg/mL) and cultured with shaking at 30°C until the absorbance at OD600 reached 0.5. Then, 1 mM IPTG was added thereto, and shake culture was performed at 30°C for an additional 5 hr. The cultured solution (50 mL) was centrifuged to collect the cells. The cells were suspended in 3 mL of 0.1 M HEPES/KOH (pH 7.4) and then homogenized by ultrasonication. The homogenate was centrifuged (15000 rpm), and the resulting supernatant was used as an E. coli crude extract. To confirm the expression of the TM2-fused SITH-1 protein, proteins contained in each crude extract were fractionated by SDS-PAGE and analyzed by Western blotting.

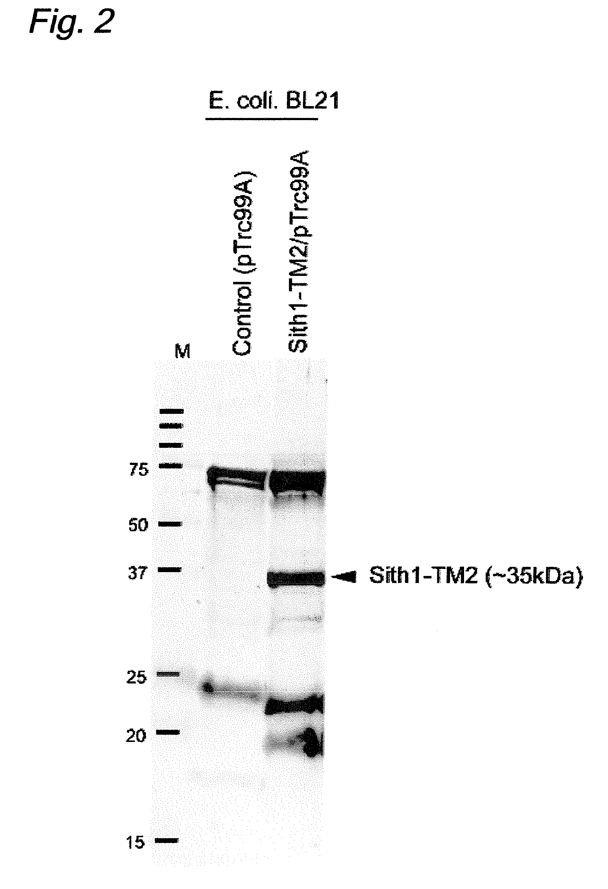

For detection of SITH-1, rabbit anti-SITH-1 antibody (i.e., anti-SITH-1 antibody (antiserum) prepared from rabbits immunized with a purified product of SITH-1 expressed in E. coli cells) and alkaline phosphatase-labeled anti-rabbit IgG antibody (BIO RAD) were each diluted 1000-fold and used. The results are shown in Fig. 2. A band of approximately 35 kDa was detected from the TM2-fused SITH-1-expressing E. coli cells. This size was substantially equal to the molecular weight (34.8 kDa) of TM2-fused SITH-1. It should be noted that E. coli crude extracts were also prepared in the same manner as shown above from E. coli BL21 carrying pTrc99A or TM2/pTrc99A (Takakura et al., (2009), FEBS J., 276: 1383-1397) after being cultured in the presence of IPTG

### Example 2: ELISA detection of anti-SITH-1 antibody in human serum

The TM2-fused SITH-1-expressing E. coli crude extract obtained in Example 1 was diluted with 0.1 M HEPES/KOH (pH 7.4) into a total soluble protein concentration of 2 mg/mL, 100 µL of which was then added to each well in a biotin plate (manufactured by Sumitomo Bakelite Co., Ltd.). The plate was allowed to stand at room temperature for 1 hr to immobilize the TM2-fused SITH-1 protein onto the biotin plate through tamavidin-biotin binding. Then, each well in the plate was washed with 0.1% Tween 20-containing TBS buffer (TBST) three times, followed by addition of a 5 µg/mL BSA/TBST solution or 5 µg/mL purified TM2/5 µg/mL BSA/TBST solution in a volume of 250 µL per well. The plate was allowed to stand at room temperature for 1 hr to block each well. Then, each well was washed with TBST three times.

Next, human serum (Human Serum pool, Cosmo-Bio, Inc.) was diluted 100-fold with PBS or with the pTrc99A-carrying E. coli crude extract (5 mg total soluble protein/mL) or the TM2/pTrc99A-carrying E. coli crude extract (5 mg total soluble protein/mL) prepared in Example 1-4. To the resulting solution, rabbit anti-SITH-1 antibody (antiserum) was added in serial dilution to give a volume ratio of 0.002, 0.001, 0.0005, or 0.00025. These human serum dilutions were added in 100 µL volumes to the plate on which the TM2-fused SITH-1 was immobilized, followed by incubation at room temperature for 1 hr.

It is expected that the antibody titer in serum of the rabbit anti-SITH-1 antibody (antiserum) is about 50 times higher compared to the SITH-1 antibody titer in serum of a patient with a typical depression (mood disorder). Accordingly, pseudo sample for serum of a patient with a typical depression can be prepared by mixing 1/50 volume of rabbit anti-SITH-1 antibody (antiserum) to a commercially available human serum (of a healthy person). Accordingly, the dilution rates of the antibody as discussed above provide samples similar to those when serum of depression patients are used after about 10 to 80 folds dilutions.

As a control, a biotin plate onto which nothing was bound was categorized into sections of two types blocking treatment, i.e., a section blocked with only BSA described above and a section blocked with BSA and TM2. In the section blocked with a solution containing tamavidin, since a part of the tamavidin in the blocking solution specifically binds to biotin on the plate, it is believed that the same state as a tamavidin-immobilized plate can be obtained. Then, human serum was diluted 100-fold with the same PBS solution or the same pTrc99A-carrying or TM2/pTrc99A-carrying E. coli crude extract (5 mg total soluble protein/mL) as used above, followed by addition of serially diluted rabbit anti-SITH-1 antibody. The resulting dilutions were added in 100 µL volumes to the control plate and incubated at room temperature for 1 hr. The rabbit anti-SITH-1 antibody was reacted with TM2-fused SITH-1 in the human serum, and then the plate was washed with TBST three times.

Then, to detect each of the rabbit anti-SITH-1 antibody bound to SITH-1 immobilized on the plate through tamavidin and the human IgG in serum which is presumed to be bound non-specifically in each well, a mixture of horseradish peroxidase-labeled goat anti-rabbit IgG antibody and peroxidase-labeled goat anti-human IgG antibody, each of which was diluted 5000-fold with TBST, was added in a volume of 100 µL per well, and the plate was left to stand for 1 hr at room temperature. Then, each well was washed with TBST three times, and peroxidase activity was detected. The activity was measured as follows: To each well, SuperSignal ELISA Pico Chemiluminescent Substrate (PIERCE) was added in a volume of 100 µL and allowed to stand for 5 min at room temperature, followed by measuring the luminescence intensity with a plate reader Infinite M200 (TECAN).

It should be noted that the data also include a luminescence intensity value measured at each concentration of rabbit anti-SITH-1 antibody for the control section sample (i.e., the section wherein the TM2 plate on which TM2-fused SITH-1 was not immobilized, but which was blocked with BSA or with both BSA and TM2 and treated with human serum containing anti-SITH-1 antibody at each concentration). The value of the control section was subtracted from the luminescence intensity value of each TM2-fused SITH-1-immobilized section. The resulting value was defined as the detected amount of anti-SITH-1 antibody contained in the serum.

Fig. 3 shows the effect of addition of E. coli homogenate extract to serum and the effect of blocking by TM2 on non-specific binding. In Fig. 3, open squares indicate the section where serum was diluted with PBS 100 fold, closed circles indicate the section where serum was diluted with an E. coli homogenate extract having only an expression vector 100 fold, and closed triangles indicate the section where serum was diluted with a TM2 expressing E. coli homogenate extract 100 fold. The vertical axis (luminescence) of each graph represents the amount of the detected anti-SITH-1 antibody while the lateral axis represents the dilution ratio of the serially diluted anti-SITH-1 antibody.

Fig. 3-1 shows the results of detection of the anti-SITH-1 antibody amount in serum without blocking by a biotin-binding protein (TM2), where open squares indicate human serum diluted with PBS, closed circles indicate human serum diluted with a pTrc99A-carrying E. coli crude extract, and closed triangles indicate serum diluted with a TM2/pTrc99A-carrying E. coli crude extract.

Fig. 3-2 shows the results of detection of the anti-SITH-1 antibody amount in serum without blocking by a biotin-binding protein (TM2), where open squares indicate human serum diluted with PBS, closed circles indicate human serum diluted with a pTrc99A-carrying E. coli crude extract, and closed triangles indicate serum diluted with a TM2/pTrc99A-carrying E. coli crude extract.

Further, the S/N ratio was calculated by the equation shown below to compare the effects of blocking and each serum dilution.

S/N ratio = Detected amount of anti-SITH-1 antibody in a section containing anti-SITH-1 antibody at each concentration/Detected amount of anti-SITH-1 antibody in a section free from anti-SITH-1 antibody
The equation shows that the detection sensitivity is higher when S/N ratio is larger.

Table 2 shows the calculated results of S/N ratio in each section.

**[Table 2]**

| Dilution ratio of anti-SITH-1 antibody | Blocking by BSA | | |
|---|---|---|---|
| | PBS | pTrc99ABL21 cell homogenate extract | TM2/pTrc99ABL21 cell homogenate extract |
| 0.002 | 1.6 | 3.8 | 6.0 |
| 0.001 | 1.7 | 3.6 | 4.7 |
| 0.0005 | 1.5 | 2.6 | 1.6 |
| 0.00025 | 1.3 | 2.2 | 0.9 |
| 0 | 1.0 | 1.0 | 1.0 |

| Dilution ratio of anti-SITH-1 antibody | Blocking by BSA and TM2 | | |
|---|---|---|---|
| | PBS | pTrc99A/BL21 cell homogenate extract | TM2/pTrc99A/BL21 cell homogenate extract |
| 0.002 | 1.7 | 5.0 | 14.3 |
| 0.001 | 1.6 | 4.7 | 14.1 |
| 0.0005 | 1.5 | 3.6 | 10.9 |
| 0.00025 | 1.3 | 2.9 | 5.5 |
| 0 | 1.0 | 1.0 | 1.0 |

| | | | |
|---|---|---|---|
| The S/N ratio in each section is normalized by the value at a dilution ratio of the anti-SITH-1 antibody of 0 (the case where the anti-SITH-1 antibody is not added, S/N=1). | | | |

The anti-SITH-1 antibody contained in the serum showed non-specific binding in most of the serum sections diluted with PBS, and a high level of luminescence intensity was also detected even in the sections free from anti-SITH-1 antibody. In contrast, in the serum sections diluted with the pTrc99A-carrying E. coli crude extract or with the TM2/pTrc99A-carrying E. coli crude extract, or in the serum sections diluted with the pTrc99A-carrying E. coli crude extract, luminescence intensity was significantly low in sections without addition of the SITH-1 antibody, and non-specific binding was dramatically reduced. The non-specific binding in the section diluted with the TM2/pTrc99A-carrying E. coli crude extract was particularly low (Figs. 3-1 and 3-2). In this case, those blocked with TM2 together with BSA (BSA+TM2) showed higher quatitativity in antibody detection in, in particular, the low concentration (dilution ratio: 0.0005 or low) range than those blocked with BSA only (Fig. 3-2).

Furthermore, it was revealed that in the section to which the pTrc99A-carrying E. coli crude extract was added and in the section to which the TM2/pTrc99A-carrying E. coli crude extract was added, the S/N ratio was increased by blocking the BSA containing TM2 to allow detection of the anti-SITH-1 antibody with high sensitivity (Table 2). In particular, in the section to which the TM2/pTrc99A-carrying E. coli crude extract was added, the increase in S/N ratio was significant.

These results indicated that non-specific binding originating from the human serum is reduced by blocking a TM2-fused SITH-1-immobilized plate with a solution containing TM2 and diluting human serum with a TM2 expressing E. coli crude extract, thereby enabling the sensitive, stable, and quantitative detection of anti-SITH-1 antibody even at a very low concentration.

### Example 3: ELISA detection of anti-SITH-1 antibody in human serum using magnetic beads

In this example, various serum diluents were studied for their effect on the detection of SITH-1 antibody at very low concentrations in the system using biotinylated magnetic beads on which TM2-fused SITH-1 was immobilized.

### 3-1. Preparation of TM2-fused SITH-1-immobilized magnetic beads

To 1 mL (30 mg beads/mL) of magnetic beads, Dynabeads M-270 Amine, (DynalBiotech), 1 mL of 10 mM NHS-Lc-Lc-Biotin (PIERCE) was added. By end-over-end mixing at room temperature for 30 min, biotin was covalently bonded to the magnetic beads through reaction between amino groups and NHS active ester groups. Then, the magnetic beads was washed twice with a 0.1% BSA/0.01% Tween 20/PBS solution, and finally suspended in PBS buffer. The resulting magnetic beads (30 mg beads/mL PBS) were used as biotinylated magnetic beads.

TM2-fused SITH-1 was expressed in E. coli cells in the same manner as shown in Example 1-4 above, and the E. coli crude extract thus prepared was diluted with 0.1 M HEPES/KOH (pH 7.4) to give a total soluble protein concentration of 5 mg/mL, to which the biotinylated magnetic beads were then added. By end-over-end mixing at room temperature for 1 hr, TM2-fused SITH-1 was immobilized on the magnetic beads through tamavidin-biotin binding. Then, the magnetic beads were washed three times with 0.2% Tween 20-containing TBS buffer (TTBS).

In addition, a crude extract of TM2-expressing E. coli cells (Takakura et al., (2009), FEBS J, 276: 1383-1397) was diluted with 0.1 M HEPES/KOH (pH 7.4) to give a total soluble protein concentration of 5 mg/mL, to which the biotinylated magnetic beads were then added. By end-over-end mixing at room temperature for 1 hr, TM2 was immobilized through tamavidin-biotin binding. After washing in the same manner as shown above, the TM2-immobilized magnetic beads thus completed (30 mg beads/mL PBS) were used for subtraction of non-specific binding inherent to serum samples.

### 3-2. ELISA analysis using magnetic beads

To commercially available human serum (Human Serum pool, Cosmo-Bio), the same rabbit anti-SITH-1 antibody as used in Example 2 was added in a 1/50 volume to prepare SITH-1 antibody-containing human serum. On the other hand, human serum free from SITH-1 antibody was used as a control.

The human serum or rabbit SITH-1 antibody-containing human serum was diluted 1000-fold with PBS or with the pTrc99A vector product-expressing E. coli crude extract or the TM2-expressing E. coli crude extract, each of which had been adjusted with 0.1 M HEPES/KOH (pH 7.4) to give a total soluble protein concentration of 5 mg/mL. To the resulting solution, BSA was further added at a final concentration of 2% (w/v). In Example 2 described above, human serum was diluted 100-fold and rabbit anti-SITH-1 antibody was then added at a volume ratio of 0.00025 to 0.002 relative to the diluted serum. However, in this Example 3-2, rabbit anti-SITH-1 antibody was added in a 1/50 volume (at a volume ratio of 0.02) to human serum and then diluted 1000-fold. Thus, the dilution factor of the rabbit SITH-1 antibody is 1/50000, which is the same as that of depression patient's serum diluted about 1000-fold.

To the serum dilutions thus prepared (1 mL each), the TM2-fused SITH-1-immobilized magnetic beads or the TM2-immobilized magnetic beads were added in 10 µL volumes and reacted by end-over-end mixing for 1 hr at room temperature, followed by washing three times with TBST. Horseradish peroxidase-labeled goat anti-rabbit IgG antibody (for detection of the rabbit anti-SITH-1 antibody bound to the SITH-1 antigen immobilized on the magnetic beads) and peroxidase-labeled goat anti-human IgG antibody (for detection of human IgG bound non-specifically to the magnetic beads) were each diluted 5000-fold with 2% BSA-containing TBST and then mixed. This peroxidase-labeled secondary antibody mixture was added in 1000 µL volumes to the magnetic beads and mixed end-over-end for 1 hr at room temperature. Then, the beads were further washed three times with TBST, and 100 µL of detection reagent (1 step ELISA ultraTMB, PIERCE) was added and reacted at room temperature for 1 min. One hundred microliter of 2 M sulfuric acid was added to stop the reaction, and the degree of color development (absorbance at 450 nm wavelength, A450) was measured with a plate reader Infinite M200 (TECAN). The measurement was performed in duplicate for each treatment section to determine a mean value.

For use as data, the A450 value measured for each serum diluent (PBS, the pTrc99A vector product-expressing E. coli crude extract, or the TM2-expressing E. coli crude extract) in the TM2-fused SITH-1-immobilized magnetic beads was processed by subtraction of the A450 value measured for the corresponding serum diluent in the TM2-immobilized magnetic beads. The results obtained are shown in Table 3.

[Table 3]

**Table 3. Effect of serum diluents on SITH-1 antibody detection in human serum**

| Serum diluent | A450 | | S/N ratio |
|---|---|---|---|
| | Serum containing SITH-1 antibody (S) | Serum free from SITH-1 antibody (N) | |
| PBS | 0.64 | 0.25 | 2.6 |
| pTrc99A-carrying | 0.14 | 0.04 | 3.5 |
| BL21 crude extract | | | |
| TM2/pTrc99A-carrying | 0.22 | 0.02 | 11.0 |
| BL21 crude extract | | | |

As shown in Table 3, the A450 value of SITH-1 antibody in human serum (S) was highest in the case of using PBS as a serum diluent, while the A450 value in serum free from SITH-1 antibody (N) was also very high, thus indicating that the S/N ratio (i.e., the ratio of the degree of color development in human serum containing rabbit anti-SITH-1 antibody to the degree of color development in serum free from rabbit anti-SITH-1 antibody) was lowest in PBS.

In contrast, when the TM2-expressing E. coli crude extract was used as a serum diluent, the A450 value in serum free from SITH-1 antibody was lowest, and hence non-specific binding could be greatly suppressed. In addition, the S/N ratio was highest when the TM2-expressing E. coli crude extract was used as a serum diluent, and the value thereof was larger than 10. This indicated that in the use of the TM2-fused SITH-1-immobilized magnetic beads, the addition of the TM2-expressing E. coli crude extract to human serum leads to a reduction in non-specific binding and high-sensitive detection of anti-SITH-1 antibody.

If depression (mood disorder) is diagnosed by quantitative determination of the SITH-1 antibody, the human serum containing rabbit anti-SITH-1 antibody and the human serum free from rabbit anti-SITH-1 antibody used in this specification can be recognized as serum of a depression (mood disorder) patient and serum of a healthy person, respectively. As shown in Table 3 above, the value of depression patient (S) is 0.64, and the value of healthy person (N) is 0.25 in the case of using PBS as a serum diluent, while the value of depression patient (S) is 0.22, and the value of healthy person (N) is 0.02 in the case of using the TM2/pTrc99A-carrying BL21 crude extract as a serum diluent. It is predicted that in a large number of clinical samples (serum), some of the sera from healthy persons show high non-specific binding, and, in contrast, some of the sera from depression patients do not clearly show a high SITH-1 antibody titer. Accordingly, as a method for detecting these differences, it is desirable that the non-specific binding (N) be low as much as possible and that the specific binding (S) can be certainly detected. From these points of view, the S/N ratio in this example is an effective index for comparing effects of each serum diluent for example.

### Example 4: ELISA detection of anti-Harpin antibody in human serum using fusion protein between Harpin and tamavidin

In this Example, a fusion protein between Harpin, which is a protein derived from a plant pathogenic bacteria (hrpZpss protein, Takakura et al., 2004, Physiol. Mol. Plant Pathol., 64: 83-89), and tamavidin 2 (TM2) was expressed in E. coli cells and was immobilized on a biotinylated microplate by tamavidin-biotin binding. The resulting Harpin plate was blocked with tamavidin 2 and was then reacted with serum (human serum containing a rabbit anti-Harpin antibody) diluted with each E. coli crude extract. Subsequently, the anti-Harpin antibody was detected using peroxidase-labeled goat anti-human IgG antibody and horseradish peroxidase-labeled goat anti-rabbit IgG antibody. The details will be described below.

### 4-1. Construction of vector for expression of fusion protein of Harpin and tamavidin 2 and E. coli expression

A gene encoding a fusion protein having a TM2 sequence located at the C-terminal side of Harpin via a linker was constructed by PCR. In order to construct a Harpin-TM2 fusion gene, primers for linking Harpin and TM2 genes via a linker (5xlinker: GGGGSGGGGSGGGGSGGGGSGGGGS) (SEQ ID NO: 18) were designed. The designed primers are summarized in Table 4.

[Table 4]

**Table 4. Primers for production of a gene encoding a fusion protein between Harpin and TM2**

| Primer Name | Sequence |
|---|---|
| HarpinNtermEcoRI-F | AAA GAA TTC atg cag agt ctc agt ctt aa |
| HarpinC-5xlink-TM2N-R | GTG AAG ATT GAA CGT CTG Agc tgc cac cgc cac cgc tgc cac cgc cac cgc tgc cac cgc cac cgc tgc cac cgc cac cgc tgc cac cgc cac cGG CTG CAG CCT GAT TGC GGG |
| HarpinC-5xlink-TM2N-F | CCC GCA ATC AGG CTG CAG CCg gtg gcg gtg gca gcg gtg gcg gtg gca gcg gtg gcg gtg gca gcg gtg gcg gtg gca gcg gtg gcg gtg gca gcT CAG ACG TTC AAT CTT CAC |

As shown in Table 4, a primer consisting of a DNA sequence encoding a C-terminal region of Harpin at the 5'-side, a linker at the center, and an N-terminal region of TM2 at the 3'-side (HarpinC-5xlink-TM2N-F, Table 4, SEQ ID NO: 27) and a primer consisting of a DNA sequence encoding the N-terminal region of TM2 at the 5'-side, the linker at the center, and the C-terminal region of Harpin at the 3'-side in the reverse direction (HarpinC-5xlink-TM2N-R, Table 4, SEQ ID NO: 26) were designed. Furthermore, a primer for the N-terminal region of Harpin, HarpinNtermEcoRI-F (including EcoRI cleavage sequence at the 5' end, Table 4, SEQ ID NO: 25) was designed.

In order to construct a Harpin-TM2 fusion gene, PCR was performed in two steps. In the first step of PCR, a Harpin region was amplified with the primers HarpinNtermEcoRI-F and HarpinC-5xlink-TM2N-R using a plasmid (Takakura et al., 2004, Physiol. Mol. Plant Pathol., 64: 83-89) in which a Harpin gene (ORF) (SEQ ID NO: 28) (encoded amino acid sequence: SEQ ID NO: 29) was integrated in pCR2.1 Vector (Invitrogen) as a template. Separately, a TM2 region was amplified with the primers HarpinC-5xlink-TM2N-F and TM2CtermBam using a plasmid obtained by integrating the TM2 gene into vector pTrc99A (WO2002/072817) as a template.

PCR was accomplished using a GeneAmp PCR System 9600 (PERKIN ELMER) in 20 µL reaction solution containing template DNA (100 ng), 10 x ExTaq buffer (2 µL, TaKaRa), 2.5 mM dNTP (1.6 µL), primers (20 pmoles each), and 5 U/µL ExTaq (0.1 µL) under the reaction conditions: 25 cycles of 94°C, 60°C, and 72°C for 30 sec each. As a result, a PCR product of about 1 kbp was obtained for the Harpin region, while a PCR product of about 0.5 kbp was obtained for the TM2 region. These PCR products were fractionated in a TAE buffer using agarose gel electrophoresis. The gel was cut out for each PCR product, and the products were collected using a QIAEX II gel extraction kit (QIAGEN). The extraction was performed according to the manual attached to the kit.

The two PCR products were used as templates to perform the second step of PCR using the primers HarpinNtermEcoRI-F and TM2CtermBam. The reaction was accomplished using a GeneAmp PCR System 9600 (PERKIN ELMER) in 20 µL reaction solution containing template DNA (100 ng), 10 x Pyrobest buffer (2 µL, TaKaRa), 2.5 mM dNTP (1.6 µL), primers (20 pmoles each), and 5 U/µL Pyrobest DNA polymerase (0.1 µL) under the reaction conditions: (94°C for 30 sec, 60°C for 1 min, 72°C for 1.5 min) x 25 cycles. As a result, a PCR product of about 1.5 kbp was obtained.

The Harpin-TM2 fusion gene obtained by PCR was cloned into vector pCR4Blunt TOPO (Invitrogen). After confirmation of the nucleotide sequence, the plasmid carrying the fusion gene was double-digested with EcoRI and BamHI, and agarose gel electrophoresis and purification were performed in the same manner as described above to collect a DNA fragment containing the fusion gene. This fragment was ligated into E. coli expression vector pTrc99A (Pharmacia), which had been digested with EcoRI and BamHI, using a Ligation Kit (TaKaRa). The ligation product was transformed into E. coli BL21 (DE3), and the nucleotide sequence was confirmed.

As described above, a vector Harpin-TM2/pTrc99A for expression of the fusion protein between Harpin and TM2 was completed. The nucleotide sequence encoding Harpin-TM2 is shown in SEQ ID NO: 30, and the amino acid sequence encoded thereby is shown in SEQ ID NO: 31.

E. coli BL21 (DE3) carrying Harpin-TM2/pTrc99A was inoculated into LB medium (50 mL) containing an antibiotic, ampicillin, (final concentration: 100 µg/mL) and cultured with shaking at 30°C until the absorbance at OD600 reached 0.5. Then, 1 mM IPTG was added thereto, and shake culture was performed at 30°C for an additional 5 hr. The cultured solution (50 mL) was centrifuged to collect the cells. The cells were suspended in 3 mL of 0.1 M HEPES/KOH (pH 7.4) and then homogenized by ultrasonication. The homogenate was centrifuged (15000 rpm), and the resulting supernatant was used as an E. coli crude extract. To confirm the expression of the TM2-fused Harpin protein, proteins contained in each crude extract were fractionated by SDS-PAGE and analyzed by Western blotting.

For detection of Harpin, rabbit anti-Harpin-1 antibody (Takakura et al., 2004, Physiol. Mol. Plant Pathol., 64: 83-89) and alkaline phosphatase-labeled anti-rabbit IgG antibody (BIO RAD) were each diluted 1000-fold and used.

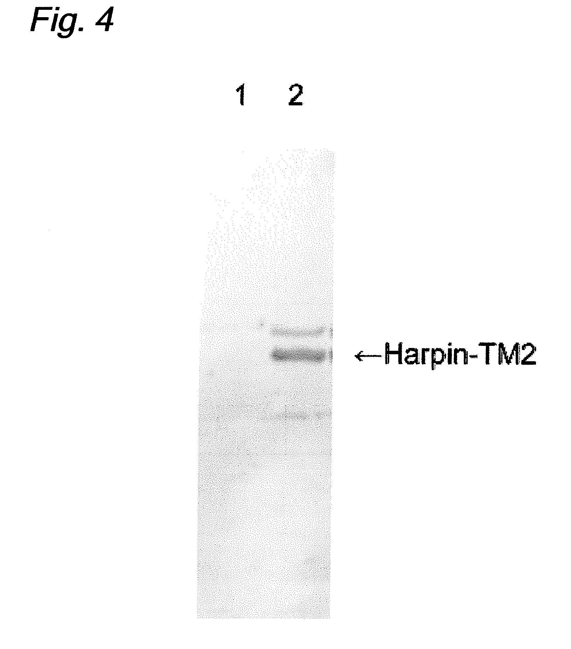

The results are shown in Fig. 4. In the E. coli homogenate extract having only an expression vector not containing an insert, no specific band was detected (Fig. 4, lane 1). In contrast, in the TM2-fused Harpin-expressing E. coli, a band migrating at approximately 50 kDa was detected (Fig. 4, lane 2). This size was substantially equal to the molecular weight (51.4 kDa) of TM2-fused Harpin.

### 4-2. ELISA detection of anti-Harpin antibody in human serum

The TM2-fused Harpin-expressing E. coli crude extract was diluted with 0.1 M HEPES/KOH (pH 7.4) into a total soluble protein concentration of 1 mg/mL, 100 µL of which was then added to each well in a biotin plate (Sumitomo Bakelite Co., Ltd.). The plate was allowed to stand at room temperature for 1 hr for immobilization of the TM2-fused Harpin through tamavidin-biotin binding. Then, each well in the plate was washed with 0.1% Tween 20-containing TBS buffer (TBST) three times, followed by addition of a 50 µg/mL purified TM2 (WO2002/072817)/0.5% BSA/TBST solution in a volume of 250 µL per well. The plate was allowed to stand at room temperature for 1 hr to block each well. Then, each well was washed with TBST three times.

Next, human serum (Human Serum pool, Cosmo-Bio, Inc.) was diluted 100-fold with PBS or with the E. coli crude extract (5 mg total soluble protein/mL 0.1 M HEPES/KOH (pH 7.4)), the pTrc99A vector product-expressing E. coli crude extract (5 mg total soluble protein/mL 0.1 M HEPES/KOH (pH 7.4)), or the TM2-expressing E. coli crude extract (5 mg total soluble protein/mL 0.1 M HEPES/KOH (pH 7.4)). After addition of 1% BSA to the resulting solution, rabbit anti-Harpin antibody was added in a volume ratio of 1/500 thereto to prepare anti- Harpin antibody containing serum. Serum free from Harpin antibody was used as a control.

These sera (100 µL each) were added to the TM2-fused Harpin-immobilized plate and were allowed to stand for reaction at room temperature for 1 hr, followed by washing with TBST three times. A horseradish peroxidase-labeled goat anti-rabbit IgG antibody (for detection of the rabbit anti-Harpin antibody bound to the Harpin antigen) and a peroxidase-labeled goat anti-human IgG antibody (for detection of human IgG bound non-specifically to the wells) were each diluted 5000-fold with 1% BSA-containing TBST and then mixed. This peroxidase-labeled secondary antibody mixture was added in 100 µL volumes to the wells. The plate was allowed to stand for a reaction at room temperature for 1 hour. Then, the plate was further washed with TBST three times, and 100 µL detection reagent (1 step ELISA ultraTMB, PIERCE) was added and reacted at room temperature for 5 min. One hundred microliter of 2 M sulfuric acid was added to stop the reaction, and the degree of color development (absorbance at 450 nm wavelength, A450) was measured with a plate reader Infinite M200 (TECAN). The measurement was performed in triplicate for each treatment section to determine a mean value. In this example, the concentration of the antibody was relatively high, and the system was relatively low in non-specific binding. Consequently, subtraction of the measured value at the section where the antigen was not immobilized was unnecessary.

Table 5 shows the results.

[Table 5]

**Table 5. Effect of serum diluents on Harpin antibody detection in human serum**

| Serum diluent | A450 | | S/N ratio |
|---|---|---|---|
| | Serum containing Harpin antibody (S) | Serum free from Harpin antibody (N) | |
| PBS | 2.26 | 0.23 | 9.7 |
| E. coli (BL21) crude | 2.13 | 0.19 | 11.3 |
| extract | | | |
| pTrc99A-carrying | 2.03 | 0.15 | 13.4 |
| BL21 crude extract | | | |
| TM2/pTrc99A-carrying BL21 crude extract | 2.16 | 0.12 | 16.8 |

As shown in Table 5, the A450 values (S) of Harpin antibody in human serum did not differ significantly among the serum diluents, whereas the values (N) in serum free from Harpin antibody as the control decreased in the order of the TM2/pTrc99A-carrying BL21 crude extract, the pTrc99A-carrying BL21 crude extract, the E. coli (BL21) crude extract, and PBS. This indicates that the TM2/pTrc99A-carrying BL21 crude extract could suppress non-specific binding in the highest degree. Regarding the S/N ratio (i.e., the degree of color development in human serum containing rabbit anti-Harpin antibody/the degree of color development in serum free from rabbit anti-Harpin antibody), the case of using the tamavidin 2-expressing E. coli extract (TM2/pTrc99A-carrying BL21 crude extract) as the serum diluent showed the highest value.

These results indicated that the dilution of human serum with an E. coli extract that has expressed tamavidin 2 suppresses non-specific binding to provide high-sensitive detection.

### SEQUENCE LISTING FREE TEXT

SEQ ID NO: 1: amino acid sequence of SITH-1

SEQ ID NO: 2: nucleotide sequence of SITH-1 ORF

SEQ ID NO: 3: nucleotide sequence of SITH-1 cDNA

SEQ ID NO: 4: nucleotide sequence of tamavidin 1

SEQ ID NO: 5: amino acid sequence of tamavidin 1

SEQ ID NO: 6: nucleotide sequence of tamavidin 2

SEQ ID NO: 7: amino acid sequence of tamavidin 2

SEQ ID NO: 8: example of linker sequence

SEQ ID NO: 9: example of linker sequence

SEQ ID NO: 10: example of linker sequence

SEQ ID NO: 11: example of linker sequence

SEQ ID NO: 12: example of linker sequence

SEQ ID NO: 13: example of linker sequence

SEQ ID NO: 14: example of linker sequence

SEQ ID NO: 15: example of linker sequence

SEQ ID NO: 16: example of linker sequence

SEQ ID NO: 17: example of linker sequence

SEQ ID NO: 18: example of linker sequence (used in Example)

SEQ ID NO: 19: nucleotide sequence of primer SITH1C-5xlink-TM2N-F

SEQ ID NO: 20: nucleotide sequence of primer SITH1C-5xlink-TM2N-R

SEQ ID NO: 21: nucleotide sequence of primer SITH1 5' EcoRI-F

SEQ ID NO: 22: nucleotide sequence of primer TM2CtermBam

SEQ ID NO: 23: nucleotide sequence of SITH-1-TM2

SEQ ID NO: 24: amino acid sequence of SITH-1-TM2

SEQ ID NO: 25: nucleotide sequence of primer HarpinNtermEcoRI-F

SEQ ID NO: 26: nucleotide sequence of primer HarpinC-5xlink-TM2N-R

SEQ ID NO: 27: nucleotide sequence of primer HarpinC-5xlink-TM2N-F

SEQ ID NO: 28: nucleotide sequence of Harpin gene (ORF)

SEQ ID NO: 29: amino acid sequence encoded by Harpin gene (ORF)

SEQ ID NO: 30: nucleotide sequence of Harpin-TM2

SEQ ID NO: 31: amino acid sequence of Harpin-TM2

## Claims

1. A method for detecting a substance in a biological sample, which comprises:
1) providing a carrier on which biotin is bound and providing a fusion protein between a protein that specifically binds to a substance to be detected and a biotin-binding protein;
2) binding the fusion protein to the carrier provided in step 1) through binding between biotin and the biotin-binding protein to produce a fusion protein-bound carrier;
3) mixing
(a) a biological sample, and
(b-i) a cell homogenate extract prepared from cells of the same species as the host cells used for expressing the fusion protein in step 1), and a biotin-binding protein, or
(b-ii) a cell homogenate extract prepared from cells of the same species as the host cells used for expressing the fusion protein in step 1), and genetically engineered to express a biotin-binding protein,
and adding the mixture to the fusion protein-bound carrier produced in step 2); and
4) detecting a substance that has bound to the protein that specifically binds to a substance to be detected in the fusion protein.

2. A method for detecting a substance in a biological sample, which comprises:
1) providing a carrier on which biotin is bound and providing a fusion protein between a protein that specifically binds to a substance to be detected and a biotin-binding protein;
2) binding the fusion protein to the carrier provided in step 1) through binding between biotin and the biotin-binding protein to produce a fusion protein-bound carrier;
3) putting a biotin-binding protein into contact with the fusion protein-bound carrier produced in step 2) to block the carrier;
4) after the blocking step in step 3), adding a biological sample to the fusion protein-bound carrier; and
5) detecting a substance to be detected that has bound to the protein that specifically binds to the substance in the fusion protein.

3. A method for detecting a substance in a biological sample, which comprises:
1) providing a carrier on which biotin is bound and providing a fusion protein between a protein that specifically binds to a substance to be detected and a biotin-binding protein;
2) binding the fusion protein to the carrier provided in step 1) through binding between biotin and the biotin-binding protein to produce a fusion protein-bound carrier;
3) putting a biotin-binding protein into contact with the fusion protein-bound carrier produced in step 2) to block the carrier;
4) after the blocking step in step 3), mixing
(a) a biological sample, and
(b-i) a cell homogenate extract prepared from cells of the same species as the host cells used for expressing the fusion protein in step 1), and a biotin-binding protein, or
(b-ii) a cell homogenate extract prepared from cells of the same species as the host cells used for expressing the fusion protein in step 1), and genetically engineered to express a biotin-binding protein,
and adding the mixture to the fusion protein-bound carrier; and
5) detecting a substance to be detected that has bound to the protein that specifically binds to the substance in the fusion protein.

4. The method according to claim 1 or 3, wherein step 3(b-i) in claim 1 or step 4(b-i) in claim 3 comprises adding a cell homogenate extract extracted from cells comprising any vector, as the cell homogenate extract.

5. The method according to any one of claims 1 to 4, wherein the biotin-binding protein is tamavidin or a variant thereof.

6. The method according to any one of claims 1 to 5, wherein the biological sample is selected from the group consisting of blood, serum, cerebrospinal fluid, saliva, sweat, urine, tear, lymph fluid, and mother's milk.

7. A carrier for detecting a substance in a biological sample, wherein
a fusion protein between a protein that specifically binds to a substance to be detected and a biotin-binding protein is bound to a carrier by binding between biotin and the biotin-binding protein, wherein
the fusion protein is bound to the carrier by a method comprising:
1) providing a carrier on which biotin is bound and providing a fusion protein between a protein that specifically binds to a substance to be detected and a biotin-binding protein;
2) binding the fusion protein to the carrier provided in step 1) through binding between biotin and the biotin-binding protein to produce a fusion protein-bound carrier; and
3) putting a biotin-binding protein into contact with the fusion protein-bound carrier produced in step 2) to block the carrier.

8. A kit for detecting a substance in a biological sample, which comprises:
A) a carrier on which a fusion protein between a protein that specifically binds to a substance to be detected and a biotin-binding protein is bound by binding between biotin and the biotin-binding protein; and
an agent for diluting a biological sample, comprising
B-i) a cell homogenate extract prepared from cells of the same species as the host cells used for expressing the fusion protein in step A), and a biotin-binding protein, or
B-ii) a cell homogenate extract prepared from cells of the same species as the host cells used for expressing the fusion protein in step A), and genetically engineered to express a biotin-binding protein; and/or
C) a blocking agent containing a biotin-binding protein.
